Europäisches Patentamt

(19) European Patent Office

Office européen des brevets

(11) Publication number: **0 221 633**

**A1**

(12) # EUROPEAN PATENT APPLICATION

(21) Application number: **86306013.3**

(22) Date of filing: **05.08.86**

(51) Int. Cl.⁴: **C 12 Q 1/68**

(30) Priority: **05.08.85 US 762574**

(43) Date of publication of application:
**13.05.87 Bulletin 87/20**

(84) Designated Contracting States:
**AT BE CH DE FR GB IT LI LU NL SE**

(71) Applicant: **Collaborative Research Inc.**
**128 Spring Street**
**Lexington Massachusetts 02173(US)**

(72) Inventor: **Donis-Keller, Helen**
**80 Park Street**
**Brookline Massachusetts 02146(US)**

(72) Inventor: **Knowlton, Robert G.**
**30 Shirley Street**
**Lexington Massachusetts 02173(US)**

(72) Inventor: **Braman, Jeffrey C**
**19 Alberta Drive**
**Hudson Massachusetts 10749(US)**

(72) Inventor: **Schumm, James S.**
**27 Morningside Avenue**
**Natick Massachusetts 01760(US)**

(74) Representative: **Shipley, Warwick Grenville**
**Michael et al,**
**VENNER, SHIPLEY & CO. 368 City Road**
**London EC1V 2QA(GB)**

(54) Genotyping by restriction fragment length polymorphisms.

(57) A DNA-based genotype test method is provided which utilizes a set of highly polymorphic RFLP DNA probes having high PIC values. The test method is able to distinguish individuals based upon their differing genetic information.

RFLP DNA probes are provided in a form suitable for use and are characterized by restriction maps.

Paternity testing and bone marrow transplant analysis are exemplified using the described test method.

EP 0 221 633 A1

## Genotyping by Restriction Fragment Length Polymorphisms

### Background

### Field of the Invention

The invention is in the fields of human genetics, diagnosis, and genetic engineering. More specifically, the invention concerns methods of distinguishing similar cells from different individuals by analysis of their genetic material using restriction fragment length polymorphisms (RFLPs). This DNA-based genotype analysis can be used to determine donor or host origin of hematopoietic cells in patients following bone marrow transplantation, for use in paternity testing or in virtually any case where the identity of an individual's cells or tissues is required.

### Description of Prior Art

A number of protein-based methods are currently used to test genetic identity. However, it often happens that a particular pair of individuals cannot be distinguished by standard methods. These include the immunological methods of red cell antigen typing also known as blood group analysis, histocompatibility locus antigen (HLA) testing and immunoglobulin isotype analysis. Another method is isoenzyme analysis which measures the electrophoretic mobility of blood enzymes Karyotype analysis can also be used.

The results of blood group typing and/or HLA testing are routinely used in paternity testing. However, these methods are not sufficient to positively identify an alleged father, rather the tests are used to try to exclude parentage in each case. The best analysis currently known is believed to give an exclusion probability of 90%, that is, there is, with current methods, a chance of about 1 in 10 that a suspect father will not be excluded even though he was indeed not the father.

Isoenzyme analysis is very limited as a method to distinguish among a variety of individuals in a population since only a few variants have been discovered.

Chromosome or karyotype analysis can distinguish male from female; males have an X and a Y chromosome while the females are characterized by the presence of two X chromosomes. Other morphological features of chromosomes can occasionally be used to characterize an individual.

Bone marrow transplantation is an important therapeutic aid used in the treatment of leukemia, severe combined immune deficiency disorders (SCIDS) and aplastic anemia. In order to follow a patient's progress after transplantation, it is desirable to be able to distinguish donor from recipient cells to document engraftment, to detect and study the presence of both donor and recipient hematopoietic cells and to monitor the patient for possible relapse.

The application of traditional methods to the specific problem of the identification of host cell origin in bone marrow transplantation studies has often been found to be inadequate; even the application of all available methods does not provide the required information in the majority of cases. Protein-based methods of distinguishing donor from recipient are usually of limited value following transplantation for the following reasons: red cell antigen typing is often not applicable because patients are often given blood support (packed red cells) from a variety of individuals, leukocyte isoenzyme analysis is difficult in the first weeks post transplant because of the lack of adequate cell numbers required for the assay. Chromosome analysis is rarely informative in sex identical cases and only permits study of actively dividing cells, and HLA analysis is usually not informative since the vast majority of transplant recipients are HLA identical with their donors.

Recent work has found polymorphism in the restriction endonuclease digests of human DNA. It is well known that there is extensive polymorphism in the DNA of the human population. Restriction endonucleases recognize specific nucleotide sequences in DNA and catalyze endonucleolytic cleavages, yielding DNA fragments of defined length. The theory underlying restriction fragment length polymorphisms (RFLPs) is described in Botstein, D., White, R., Skolnick, M. and Davis, R.W. (1930) Am. J. Hum. Genet. 32: 314-331 and White, R. Leppert, M., Bishop, D.T., Barker, D., Berkowitz, J., Borwn, C., Callahan, P., Holm, T. and Jerominski, L. (1985) Nature 313: 101-105 where it has been proposed to use RFLPs as genetic markers to construct a genetic linkage map of the human genome. The proposal contemplates identifying polymorphic loci by Southern blotting using restricted DNA and single strand cDNA probes, testing the loci for linkage relationships in human pedigrees, and arranging the loci into linkage groups to form a genetic map. The characterization of a highly polymorphic human locus revealed by a DNA probe, pAW101, has been described by Wyman, A.R. and White, R. (1980) Proc. Natl. Acad. Sci. USA 77: 6754-6758 and DeMartinville, B., Wyman, A.R., White, R. and Franke, U. (1982) Am. J. Hum. Genet. 34: 216-226. Recently the use of such RFLP probes has been applied to bone marrow transplantation studies (Schubach, W.H., Hackman, R., Neiman, P.E., Miller, G. and Thomas, E.D. (1982) Blood 60: 180-187; Minden, M.D., Messner, H.A. and Belch, A. (1985) J. Clin. Invest. 75: 91-93; and Witherspoon, R.P., Schubach, D.T., Neiman, P., Martin, P. and Thomas, E.D. (1985) Blood 65: 1172-1174). The use of the DNA probe pAW101 to determine donor or host origin in the cells of patients undergoing bone marrow transplanation is described in Schuback, et al. (1982) _supra_ and Minden, et al. (1985) _supra_. The DNA probe pAW1C1 and five other probes (one 3 allele locus and four 2 allele loci revealed) were also used by Ginsburg, D., Antin, J.H., Smith, B.R., Orkin, S.H. and Rappeport, J.M. (1985) J. Clin. Invest. _75_: 596-603 to distinguish donor from recipient cells in patients after bone marrow transplantation. However, the major limitation is that only one highly polymorphic RFLP probe was used and there is a limitation as to accuracy of the results obtained. Even in combination with a number of poorly informative DNA probes it is not always possible to make an unambiguous determination of donor or patient-specific cells (Ginsburg, et al. (1985) _supra_): this is particularly difficult in the cases of mixed cell populations.


Summary of the Invention

It is an object of the present invention to provide highly polymorphic RFLP DNA probes which have the capacity to distinguish individuals based upon their differing genetic information.

It is a further object of this present invention to provide identity test methods using highly polymorphic probes.

Another object of this present invention is to create a bone marrow test method using a set of RFLP probes capable of determining genetic identity in transplant cases.

It is an additional object of the present invention to use a set of RFLP probes which would allow exclusion of parentage probability in paternity determinations.

According to the invention, probes as follows:

> Lam4-427
> Lam4-1214
> Lam4-123
> Lam4-355
> Lam4-159

Lam4-966
Lam4-368
Lam4-744
Lam4-1065
Lam4-962

have been isolated and are provided from clones permitting
identification and use as diagnostic tools. The probes of this
invention are identified herein.

Many of the disadvantages of the prior arts methods are overcome by
the present invention which utilizes a number of specific, highly
polymorphic RFLP DNA probes in a method to distinguish
individuals based upon their differing genetic information. This
type of identity test has a number of applications such as
paternity testing, bone marrow transplant analysis or in
virtually any circumstance in which individuals need to be
distinguished from other individuals. The use of the sets of
highly polymorphic RFLP probes from a number of human genetic
locations described herein makes possible the unambiguous and
quantitative determination of genetic identity in virtually all
bone marrow transplant cases. Further, such a set of probes
would allow the exclusion of parentage probability in greater
than 99% of paternity determinations.

Brief Description of the Drawings and Tables

The above and other features of the invention will
become apparant to those skilled in the art from the
following description, taken in connection with the
accompanying drawings wherein:

Table 1 contains the characteristic features of 10 RFLP
DNA probes of this invention used in carrying out the tests of
this invention. All probes consist of bacteriophage lambda
Charon 4A with human genomic DNA inserts 11.0-17.0 Kilobases
(Kb) in length. Locus haplotype refers to the observed
alternative restriction fragment length patterns that are
inherited in a Mendelian fashion. Locus haplotypes determined from the Utah kindred 1331 (Camden Cell Repository, White, et al. (1985) Utah) are listed and serve to identify the respective probes. Restriction enzymes that gave similar relative fragment length differences among individuals are termed related polymorphisms; if as least three restriction enzymes gave such related patterns, the polymorphism is characterized as a DNA rearrangement type. Restriction enzyme abbreviations: L = BamHI, Bc = DclI, Bg = BglII, E = EcoRI, H = HindIII, Ha = HaeIII, Hc = HincII, M = MspI, Pv = PvuII, T = TaqI and X = XbaI;

Table 2 contains a linear map with the intervals between the sites (in kilobases) for each probe. Restriction maps were determined according to the method of Rackwitz, H., Zehetner, G., Frischauf, A. and Lehrach, H. (1984) Gene 30: 195-200;

Tables 3A - 3J contain panels of DNA from unrelated individuals hybridized with RFLP probes. Human genomic DNA purified from leucocytes of unrelated induviduals (A-W) was cleaved with the restriction endonucleases indicated and size fractionated by electrophoresis through 0.8% agarose. The genomic DNA was then transferred to nylon membranes and hybridized with nick-translated 32P labeled RFLP DNA probes. Autoradiography of the hybridized membranes was done for 1-4 days at -70oC. Restriction fragment length markers (in kilobases, Kb) are indicated in each Tables 3B - 3J and Tables 3A - 3J which aid in identifying specific useful probes on this invention are described below:

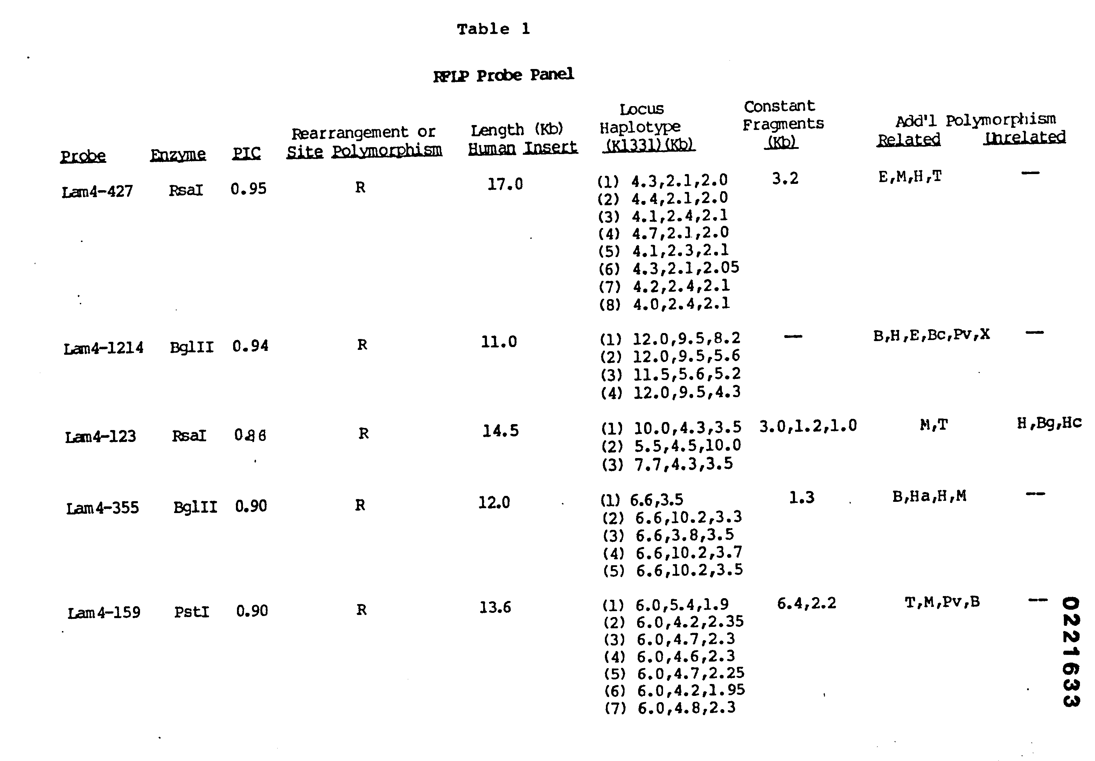

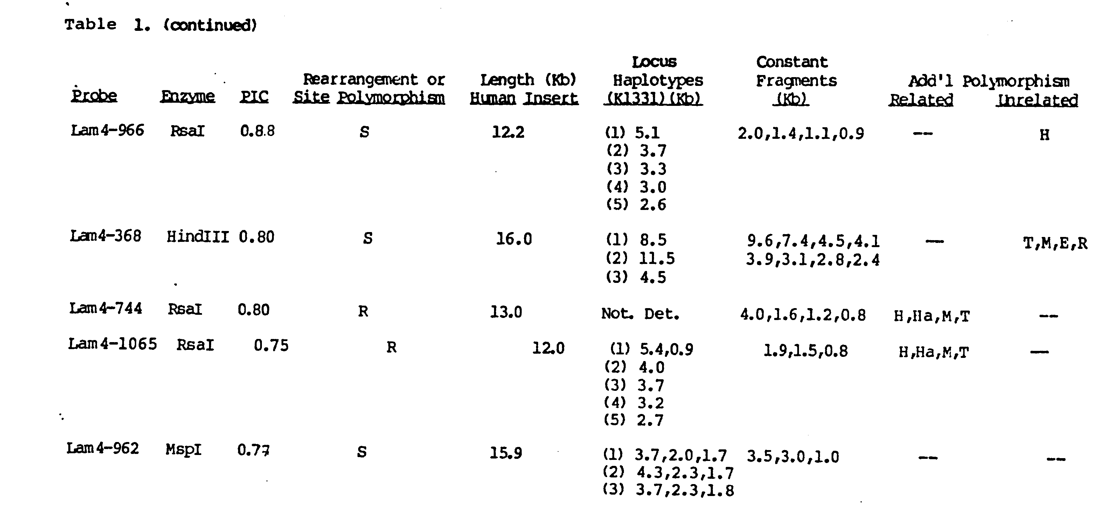

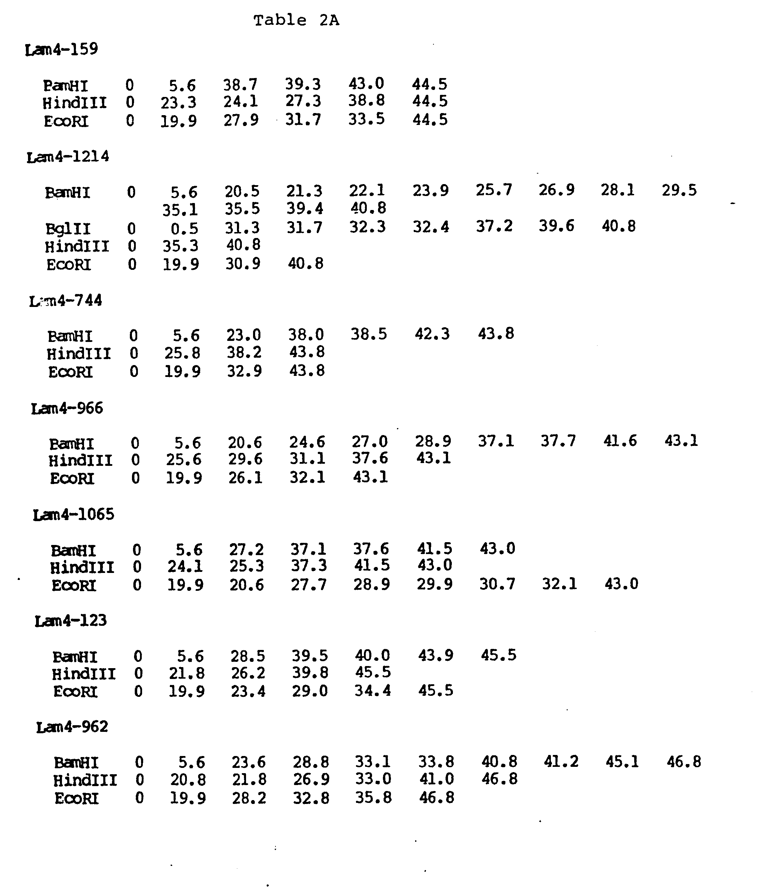

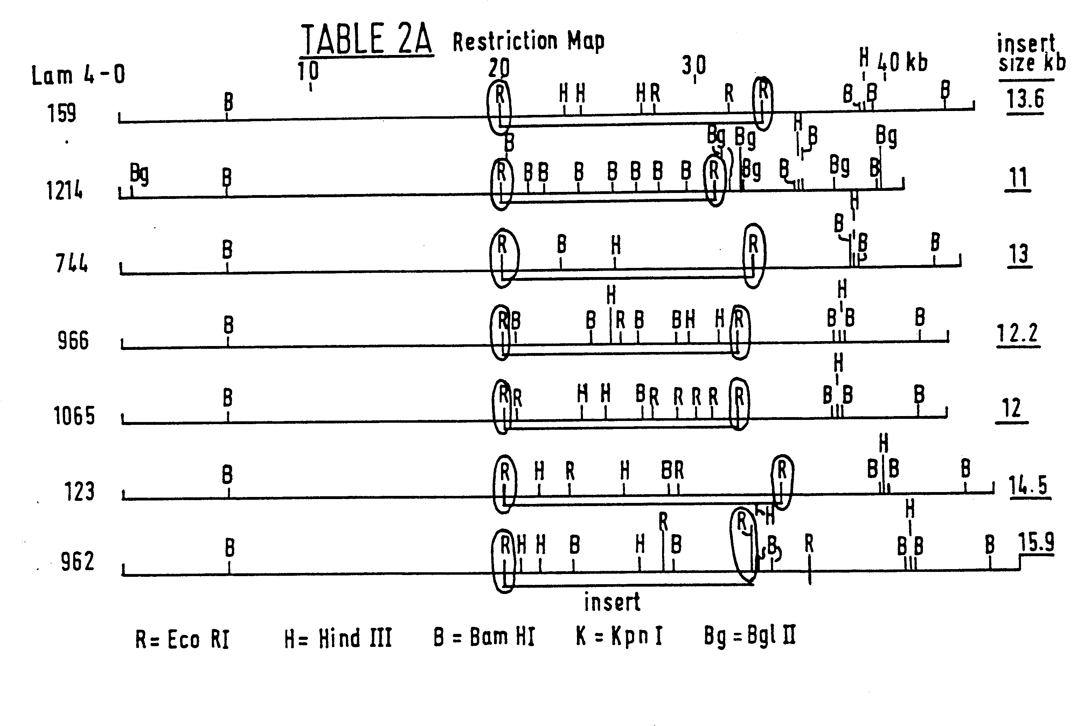

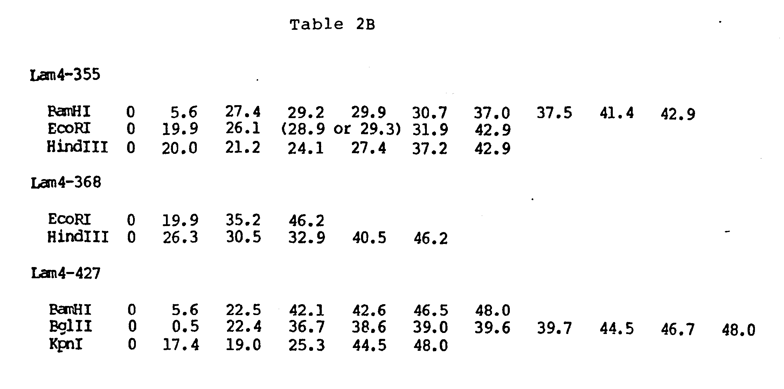

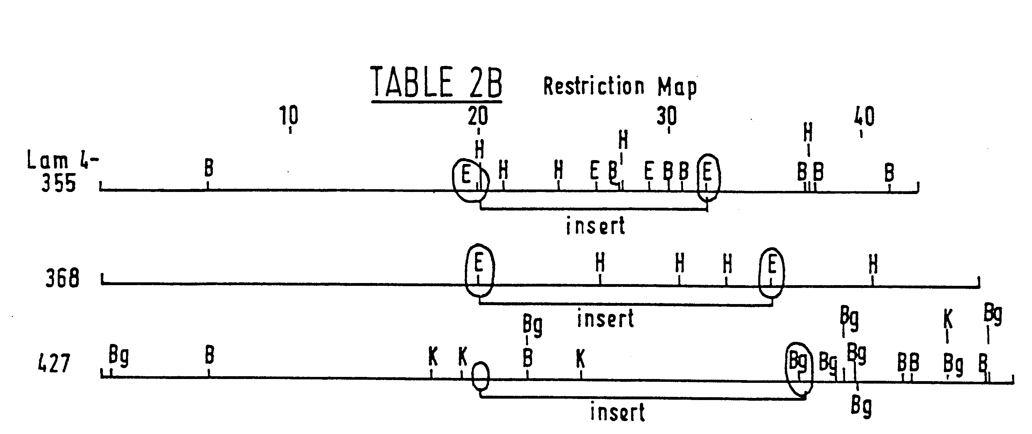

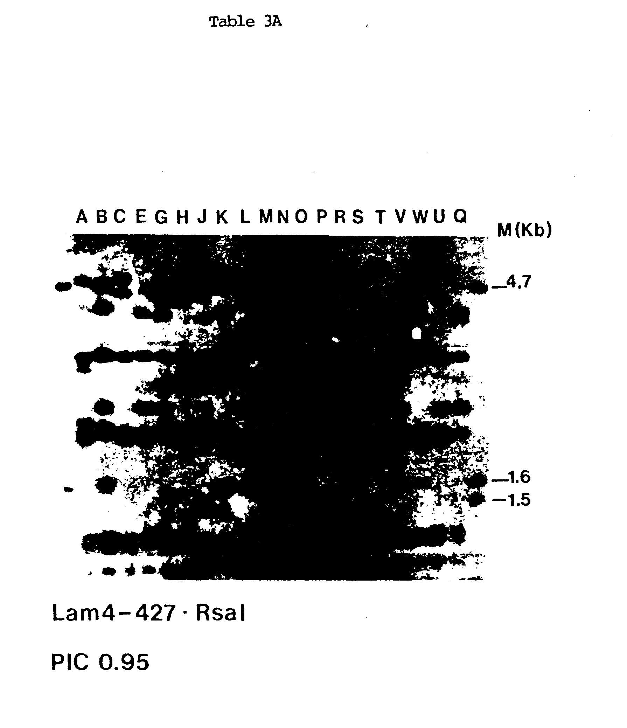

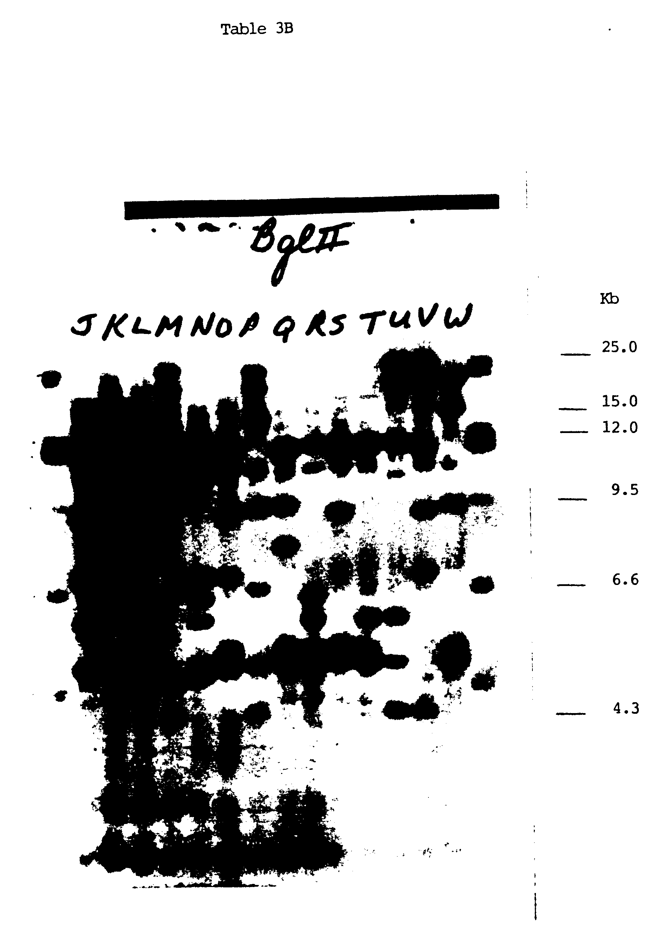

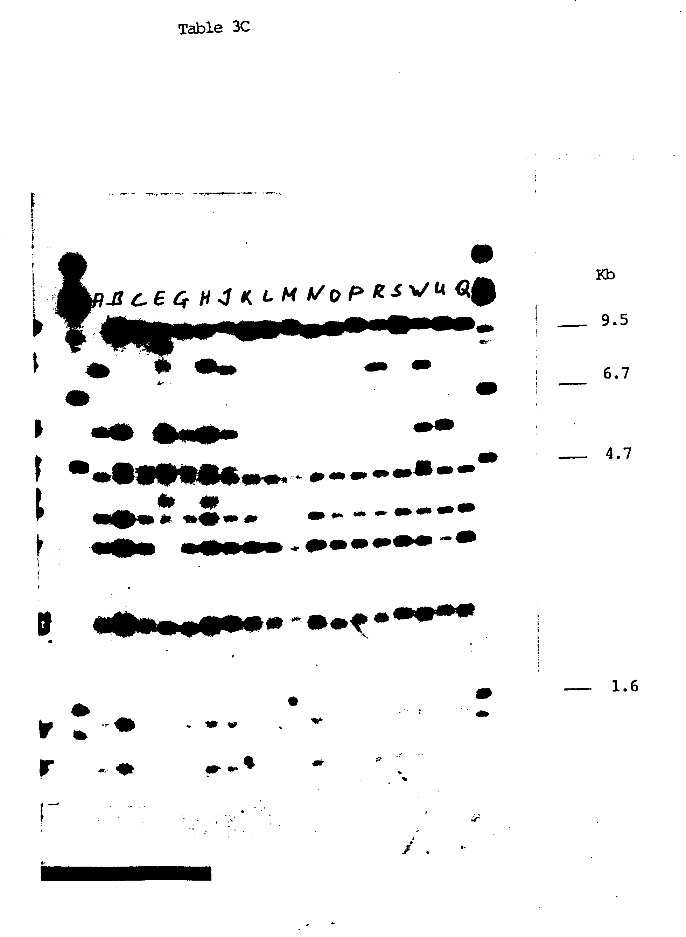

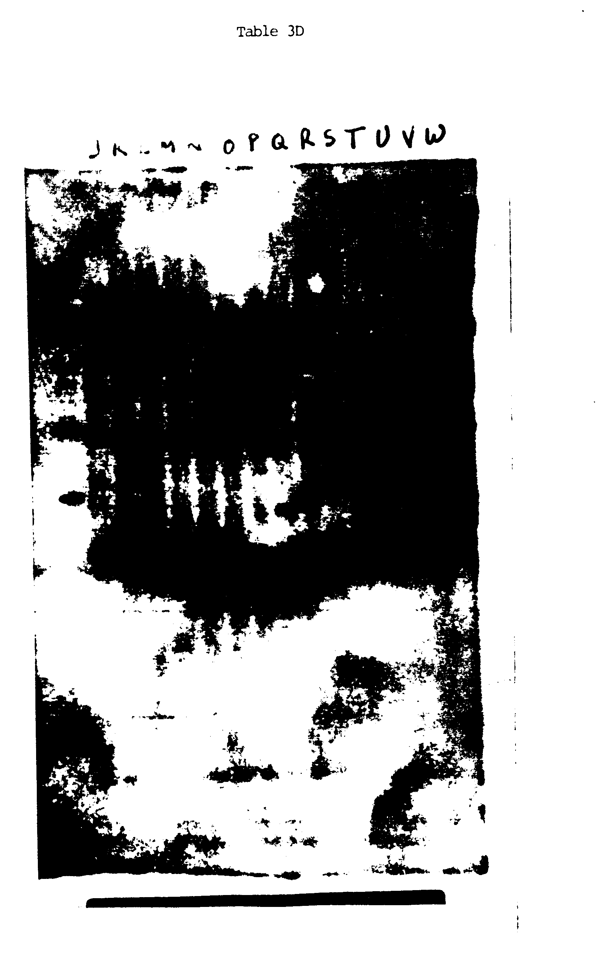

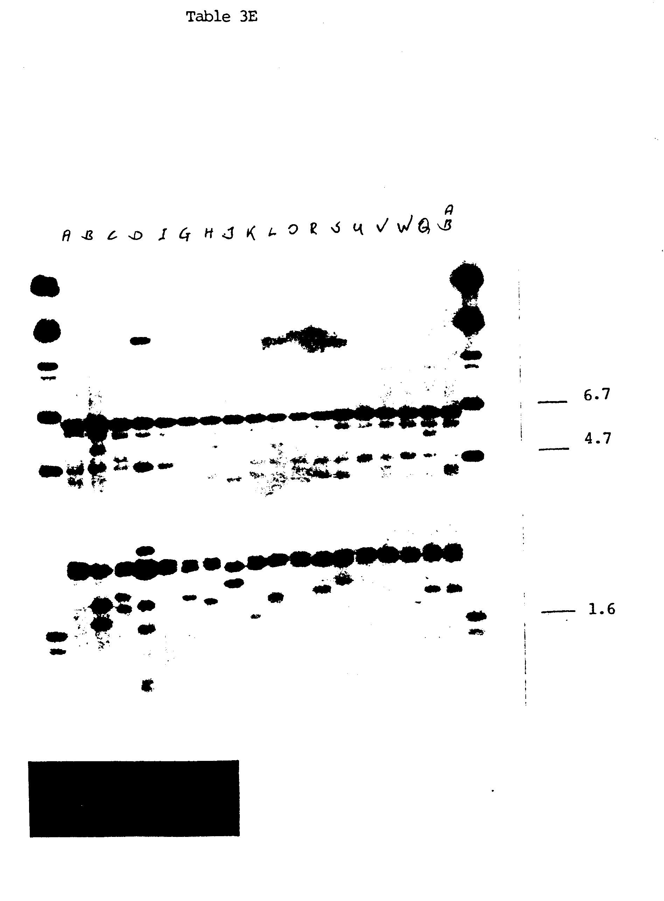

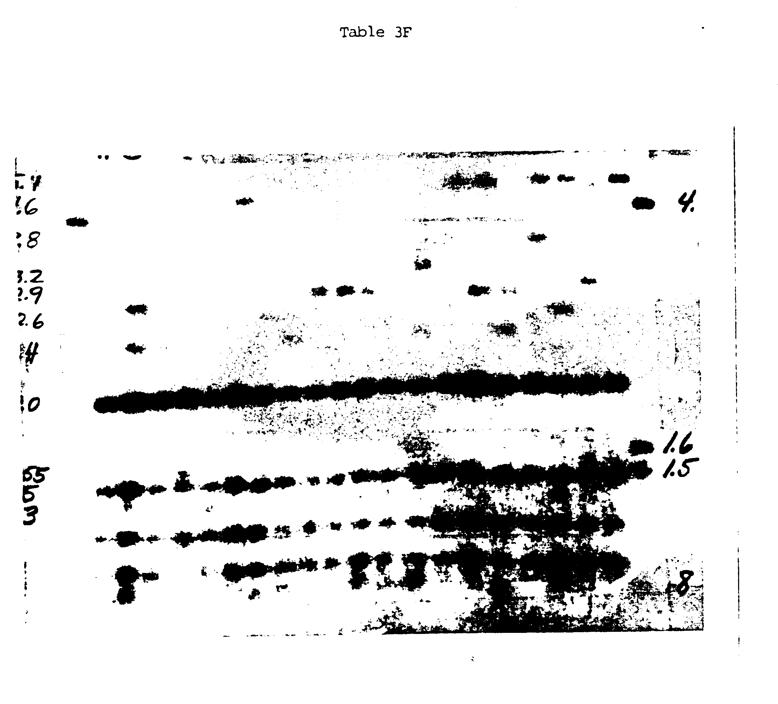

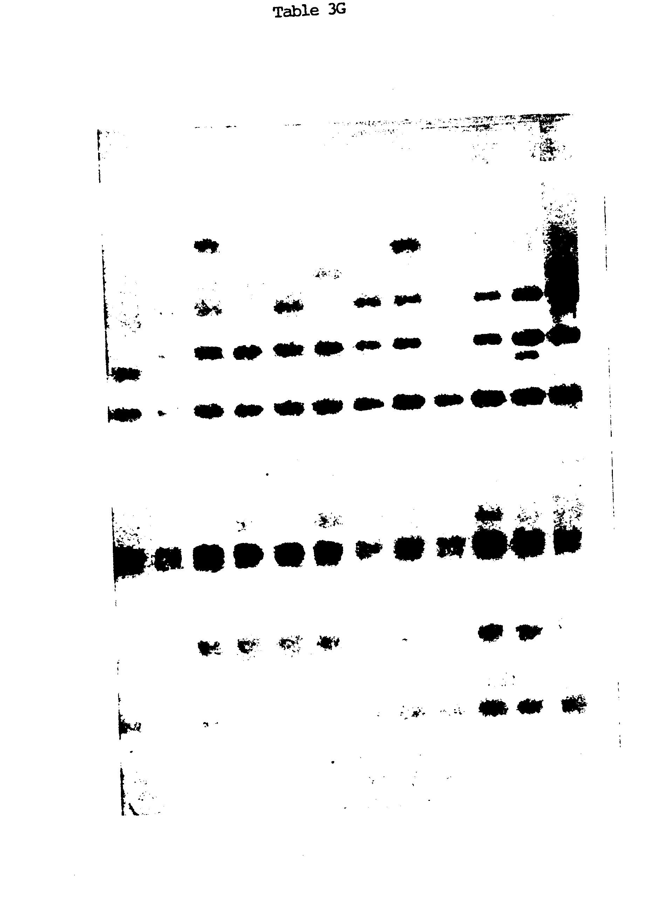

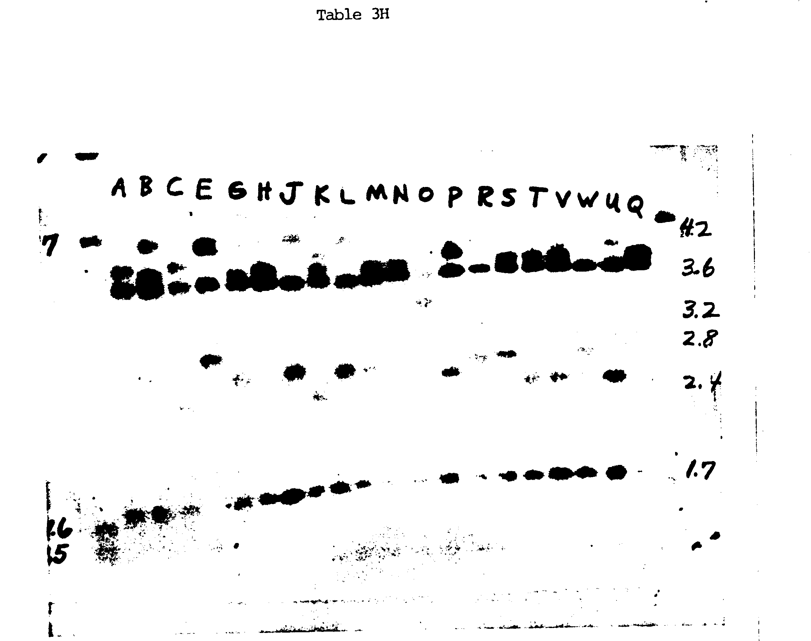

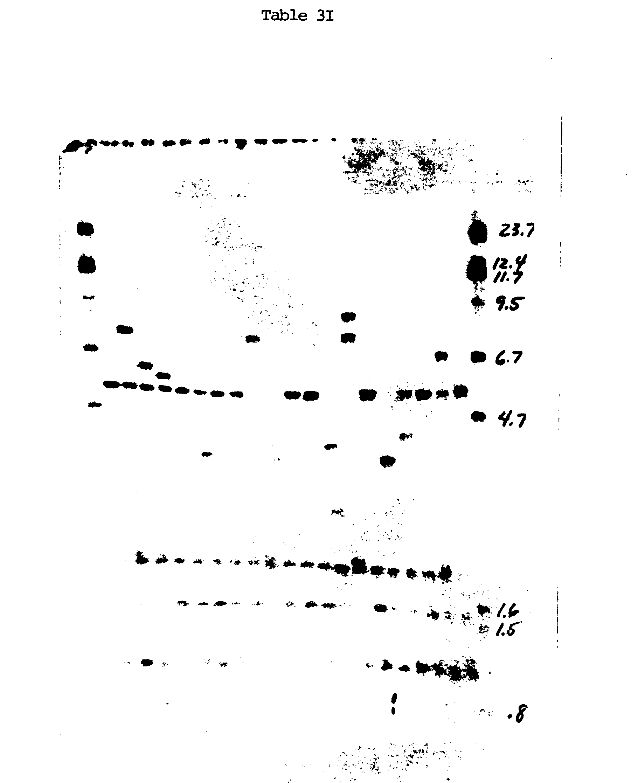

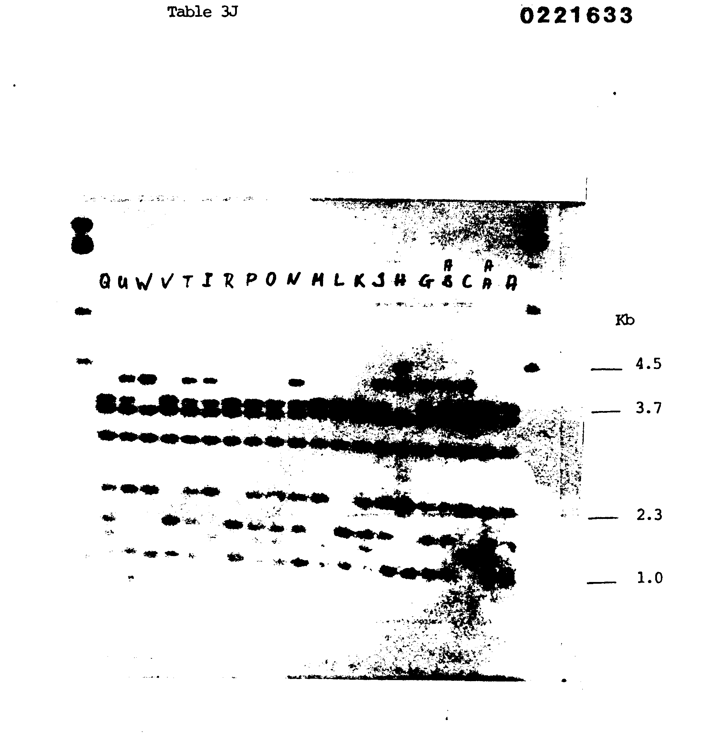

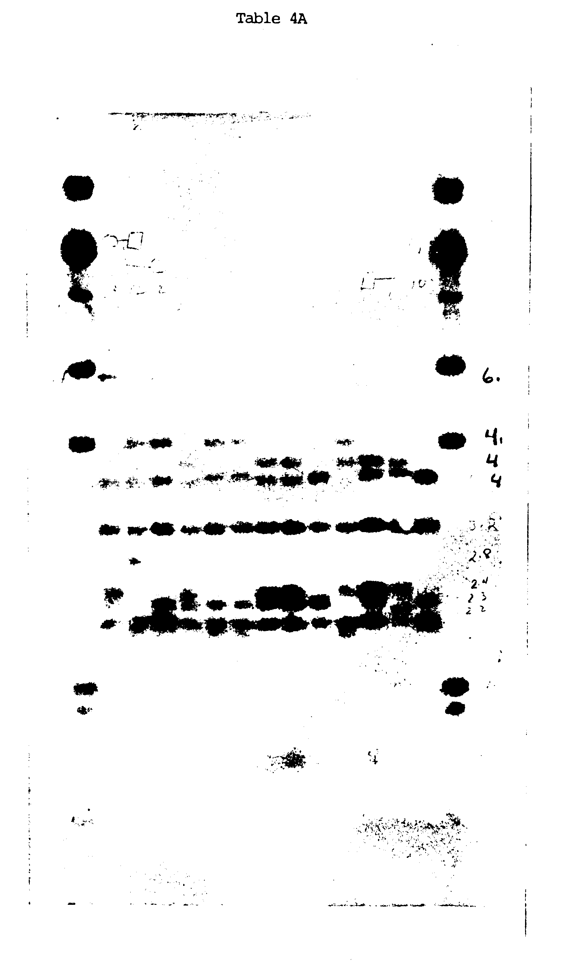

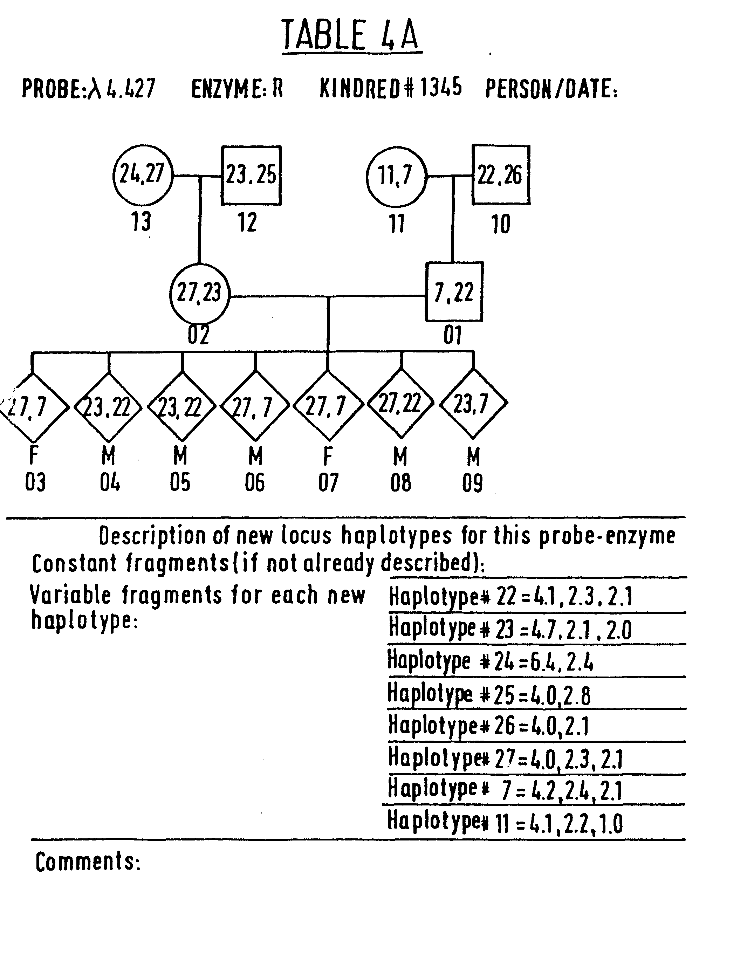

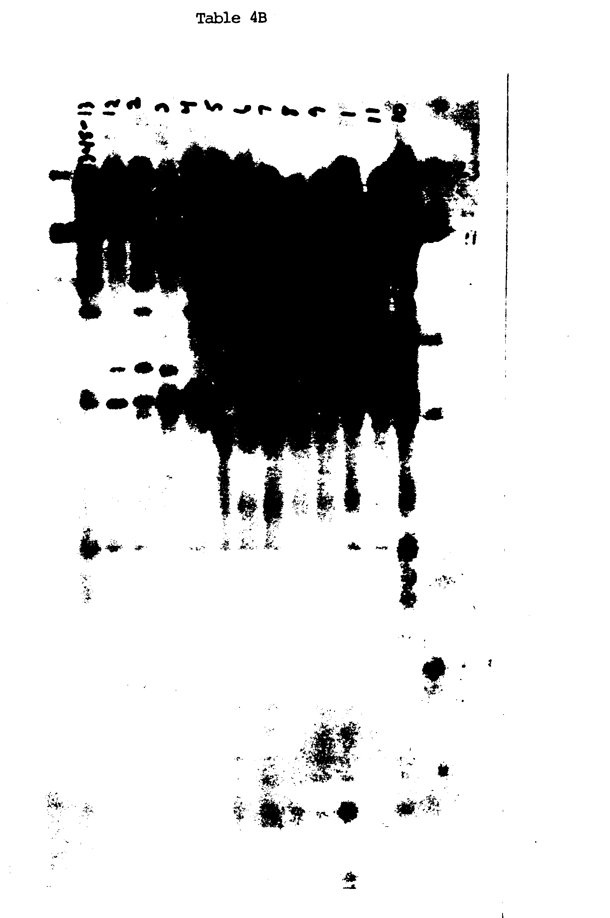

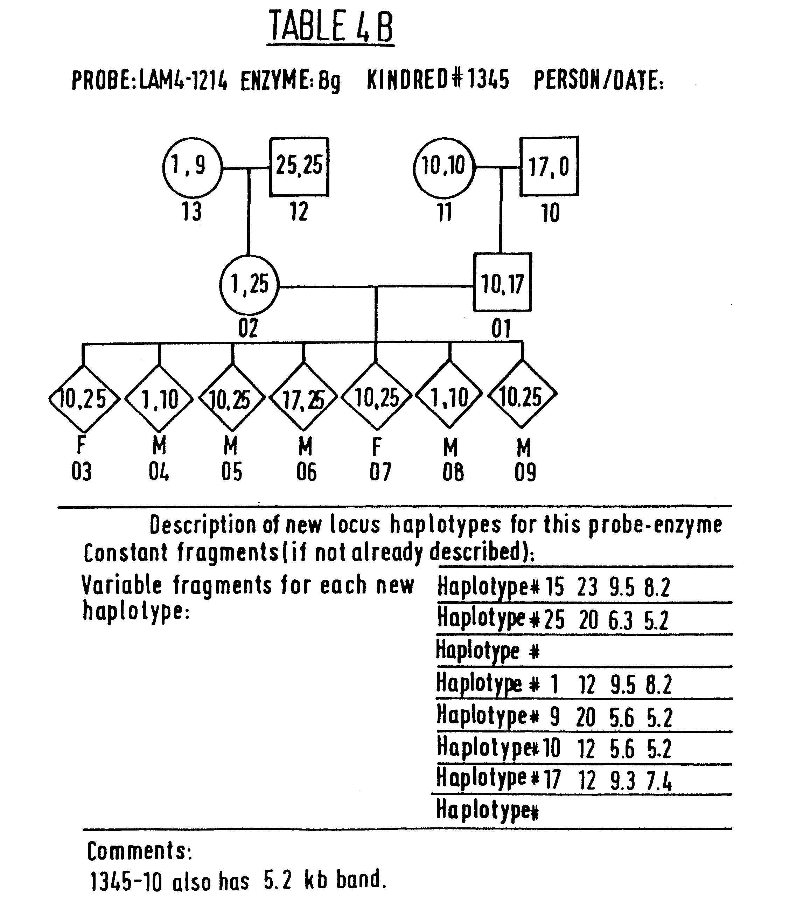

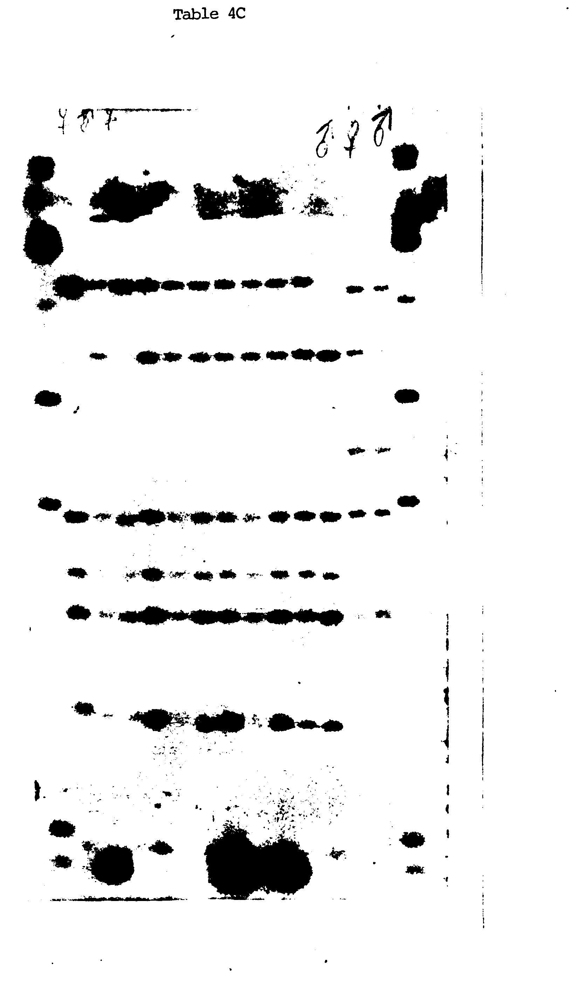

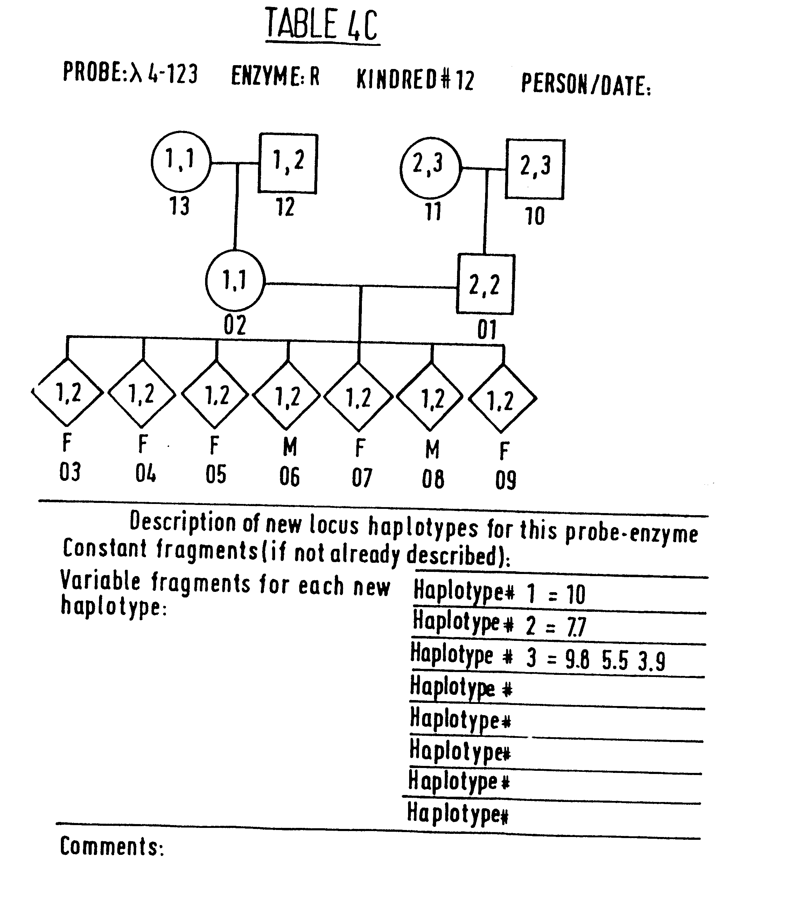

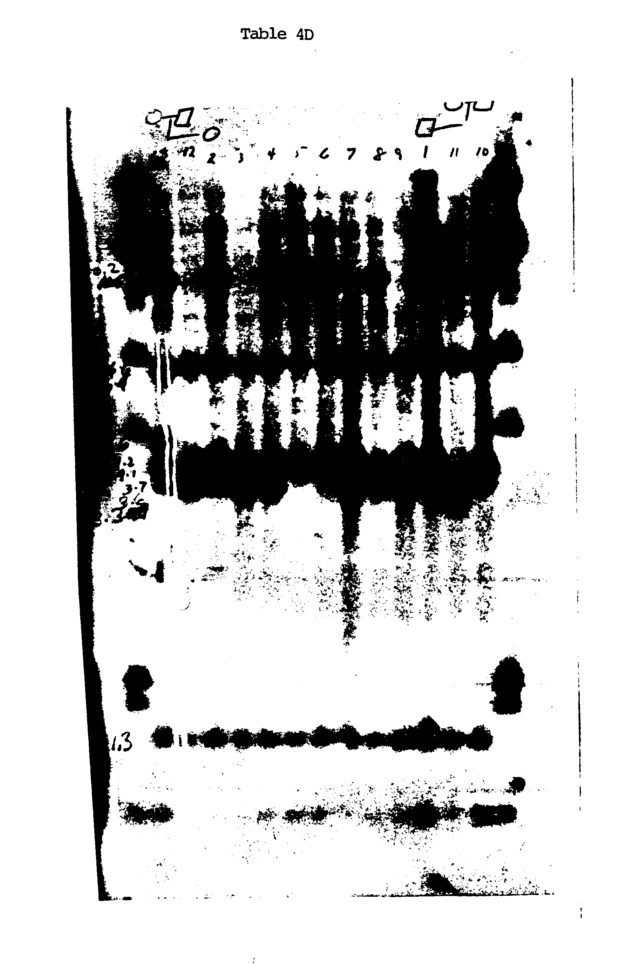

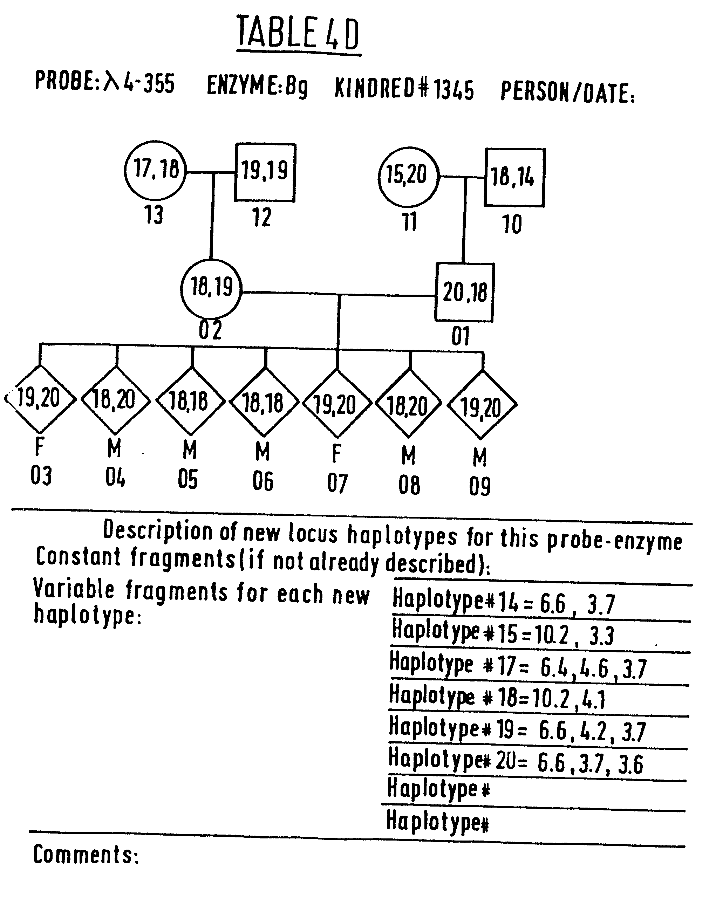

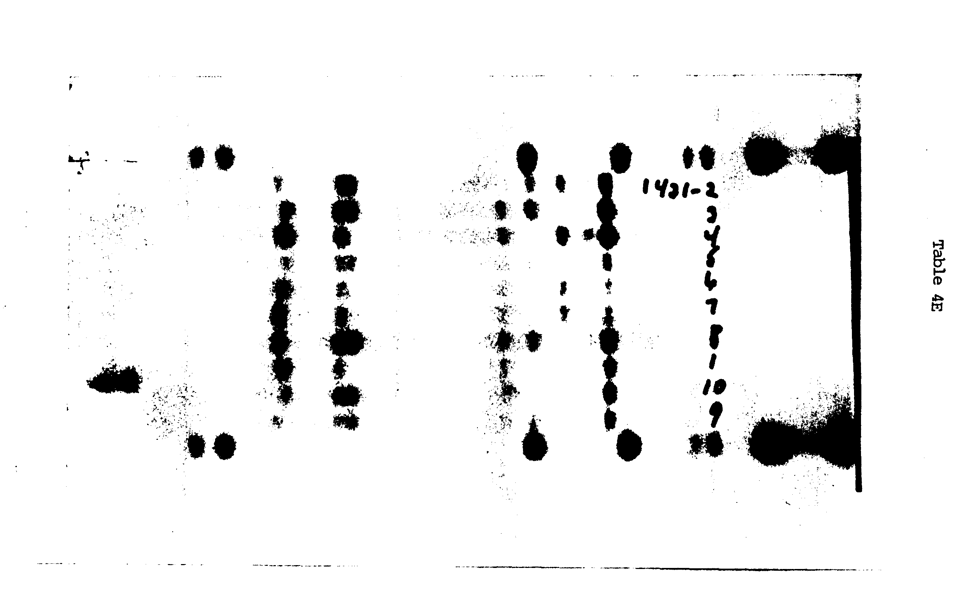

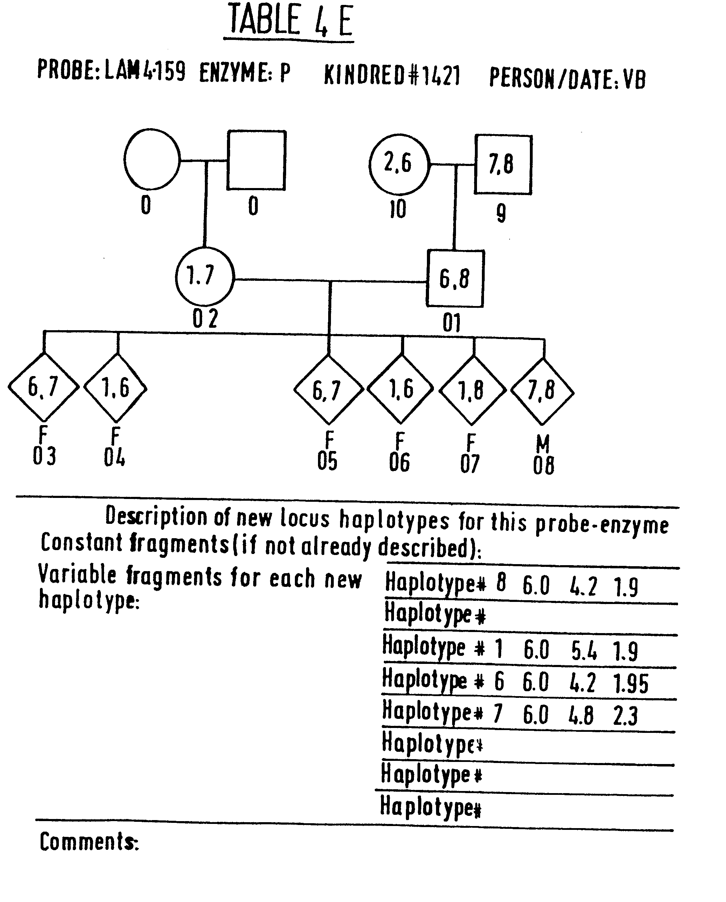

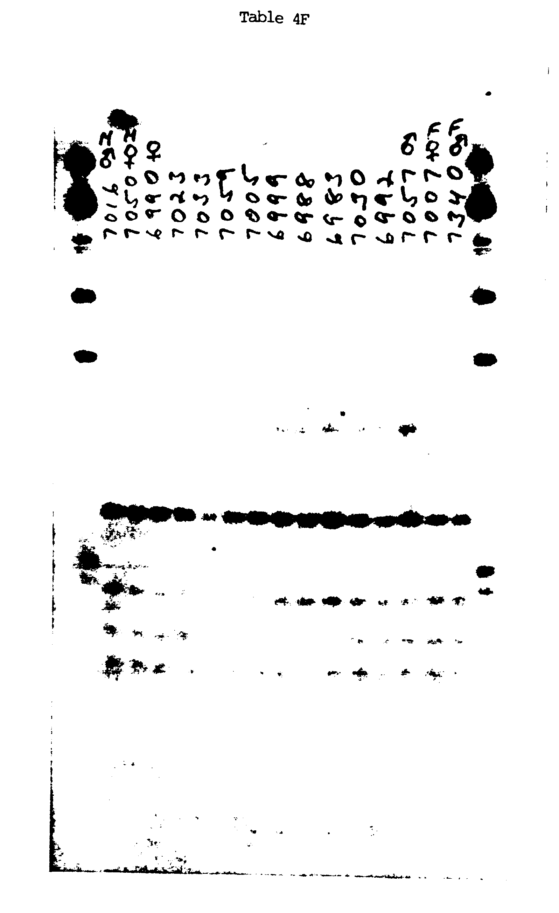

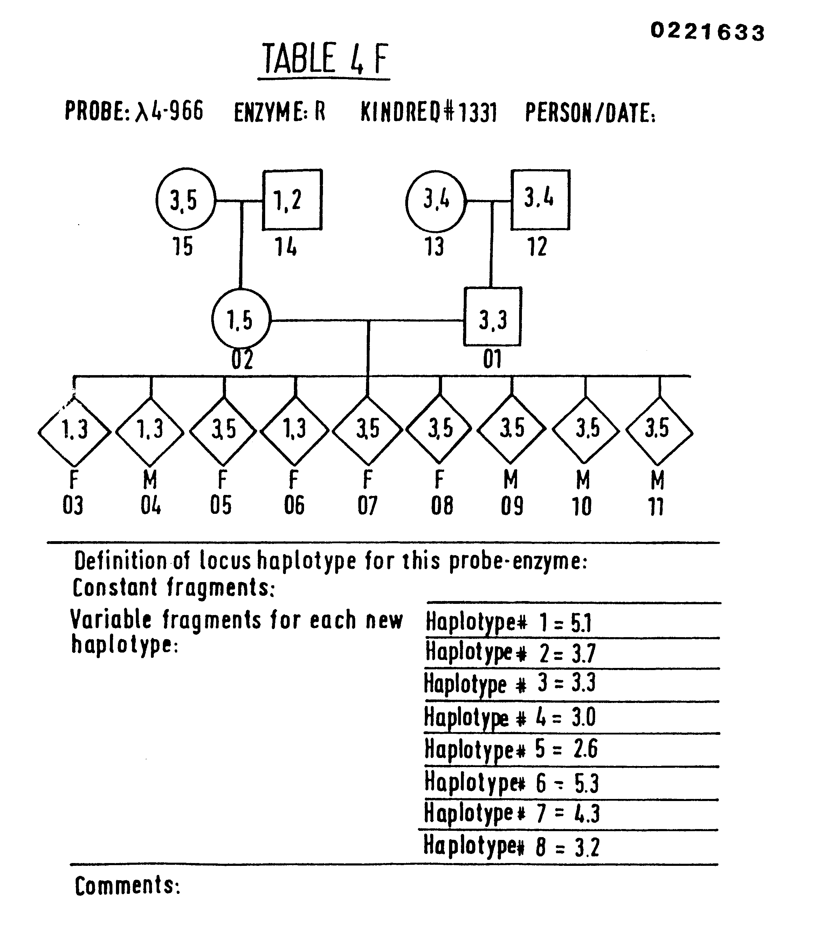

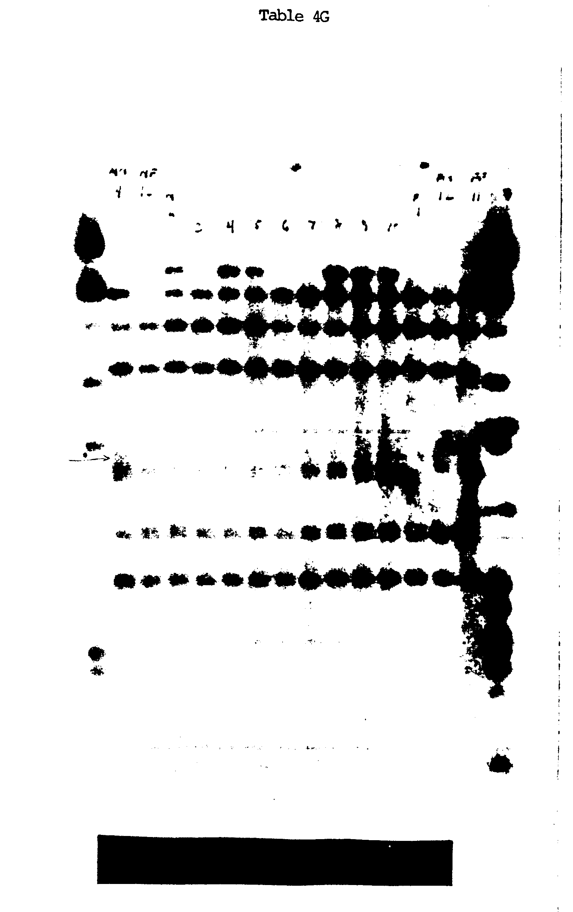

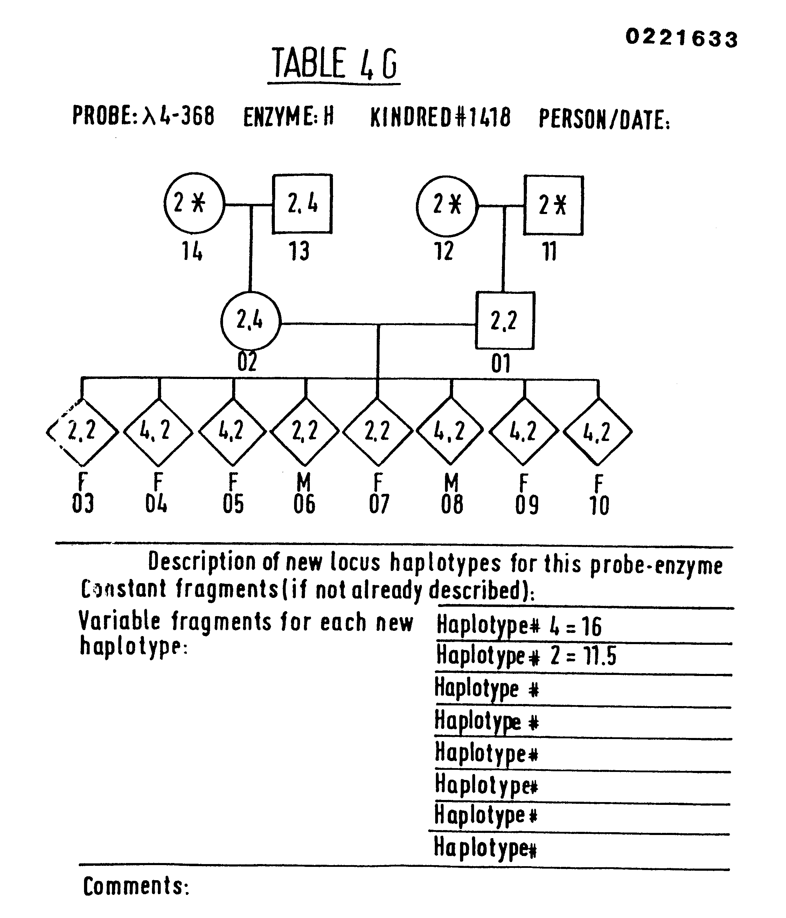

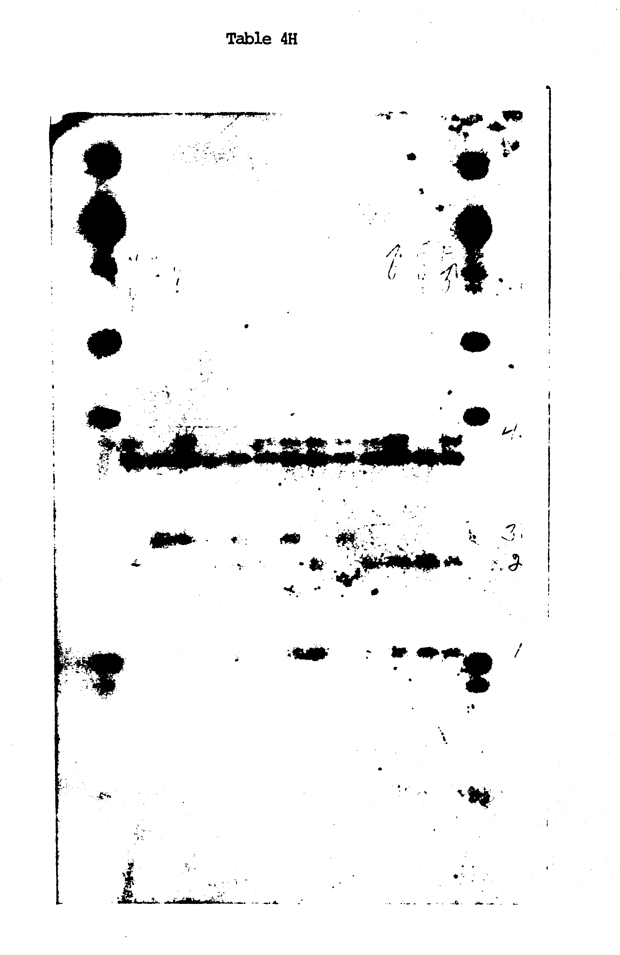

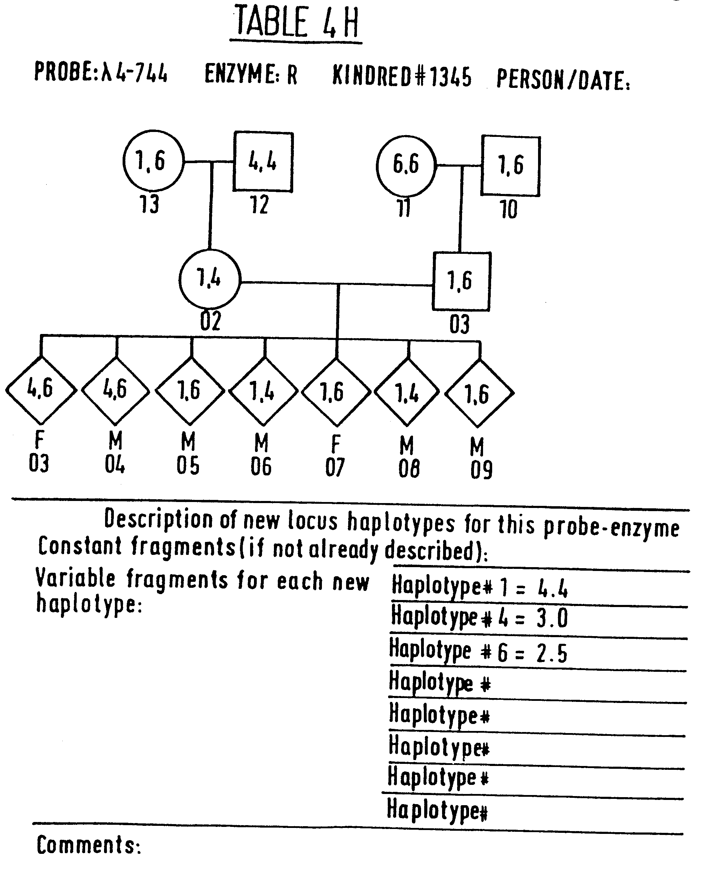

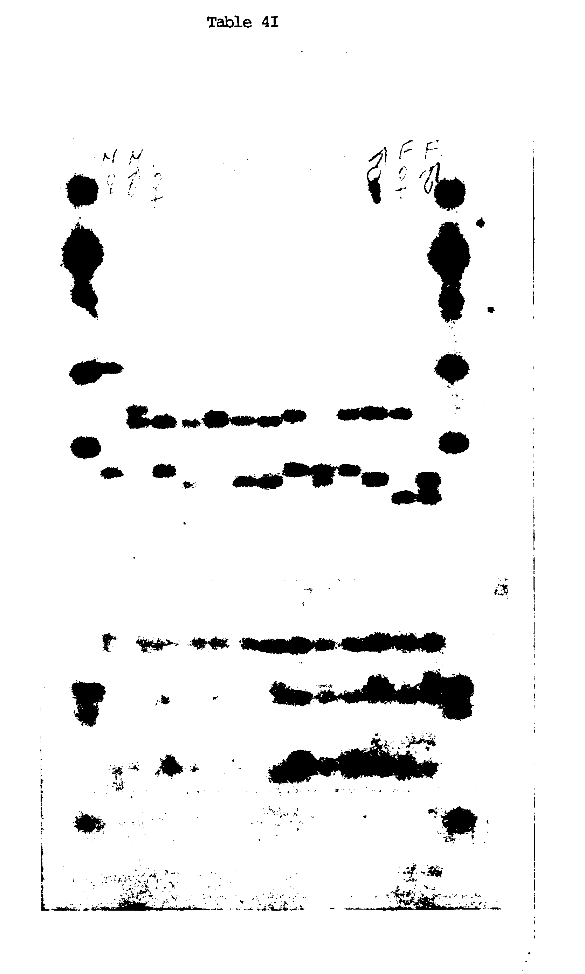

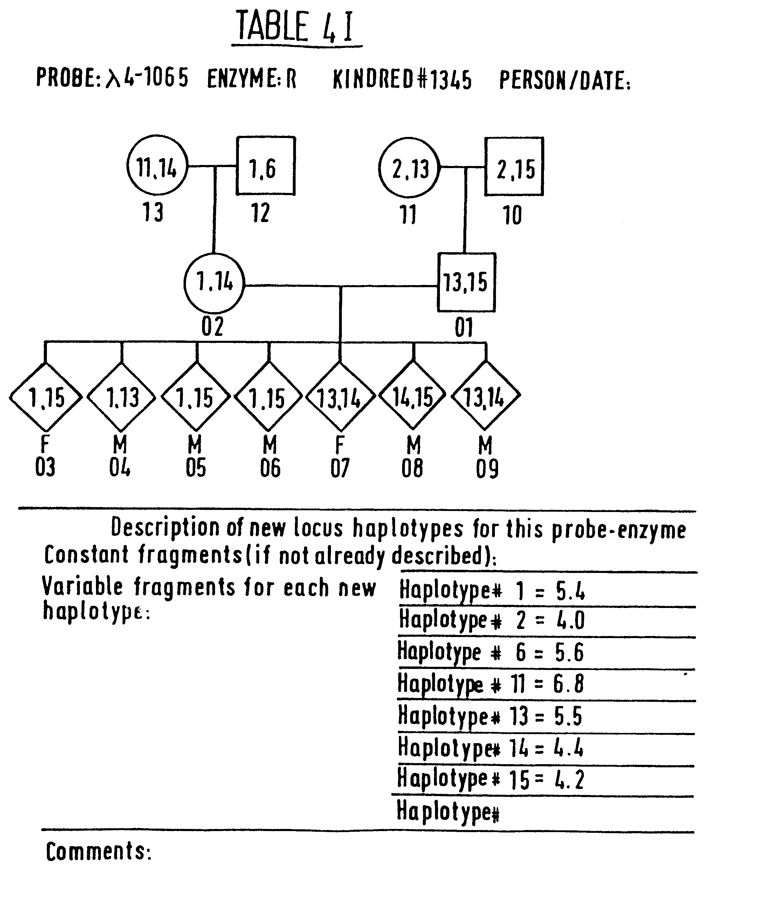

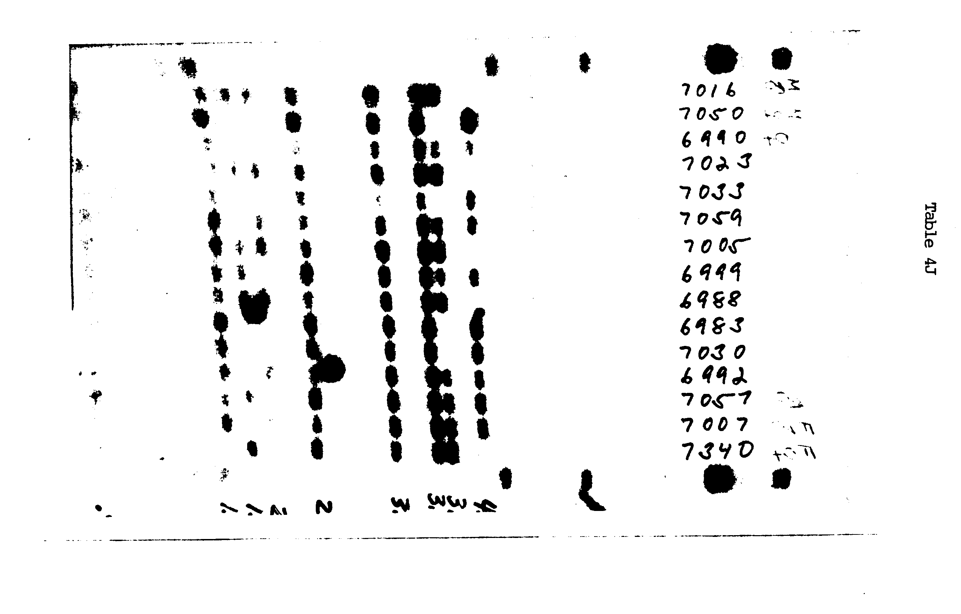

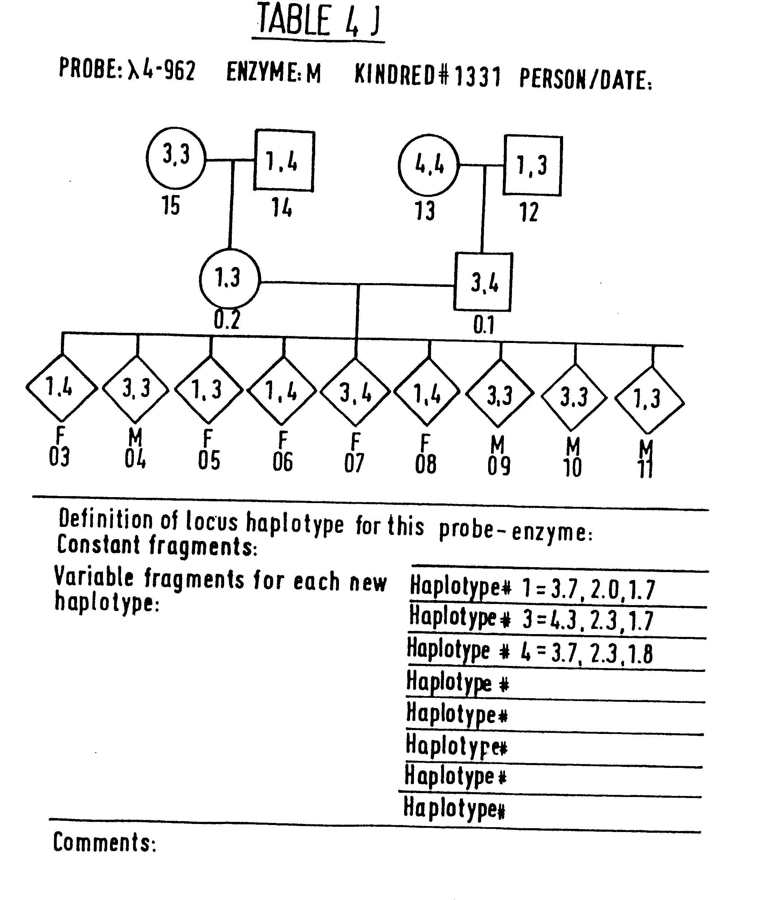

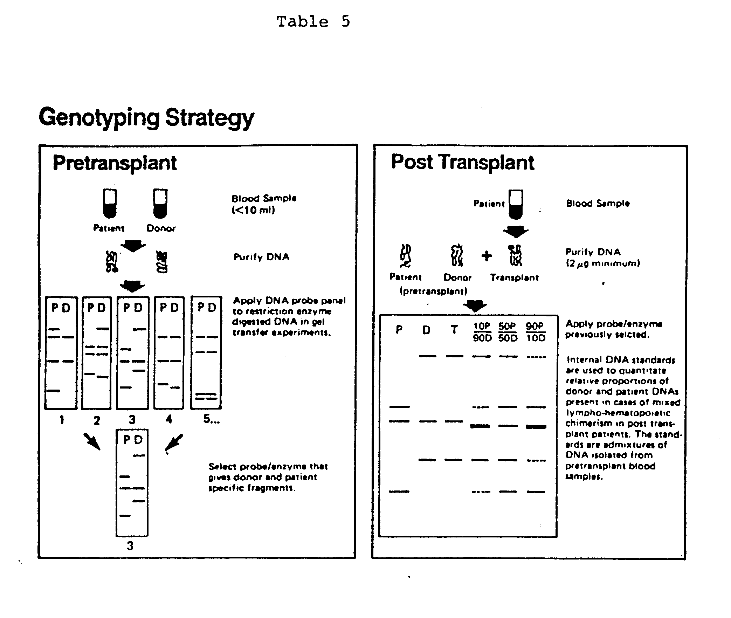

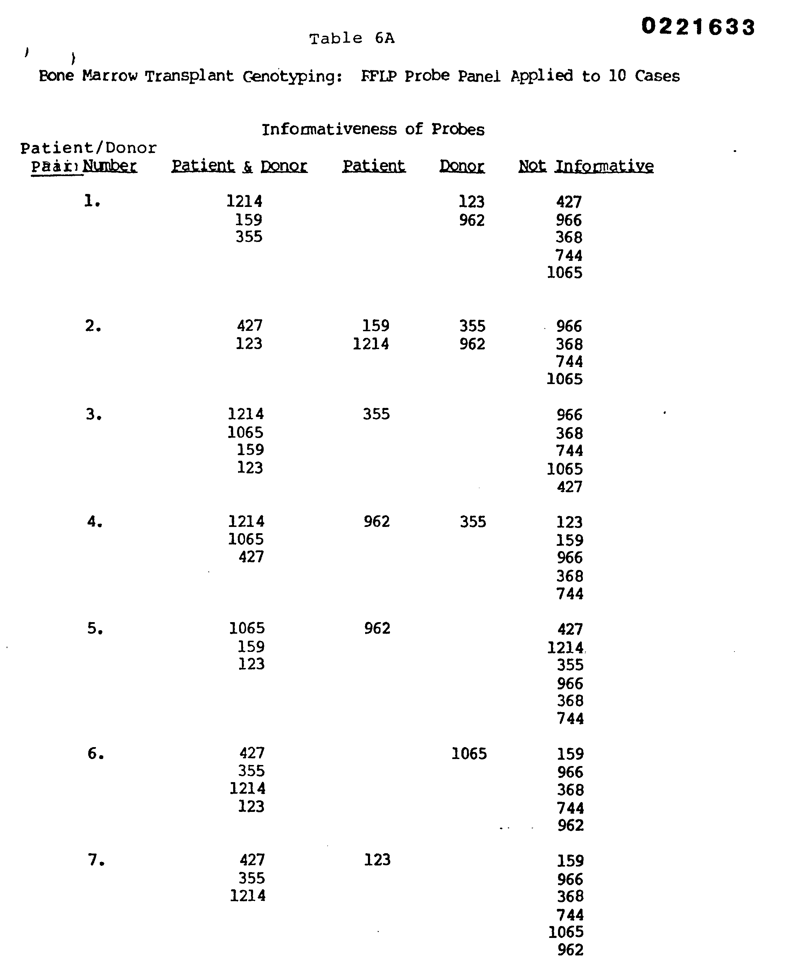

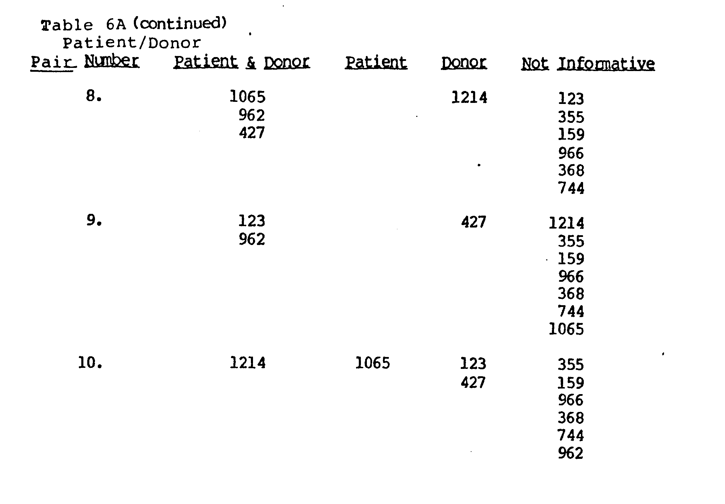

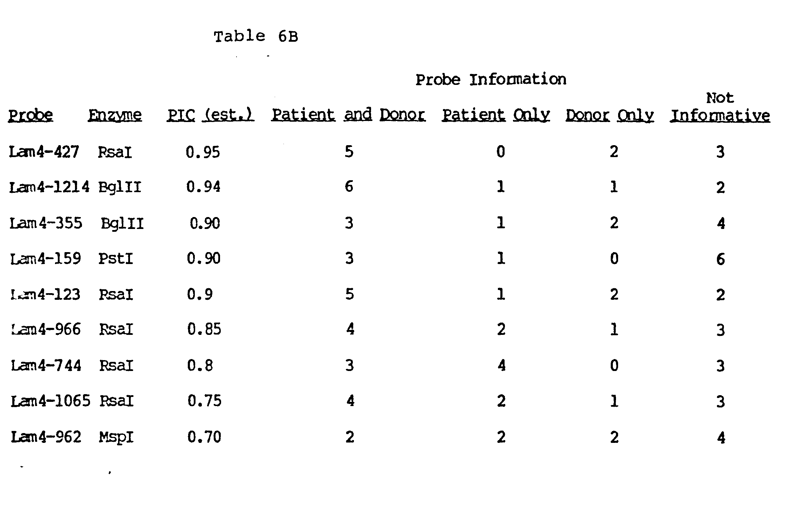

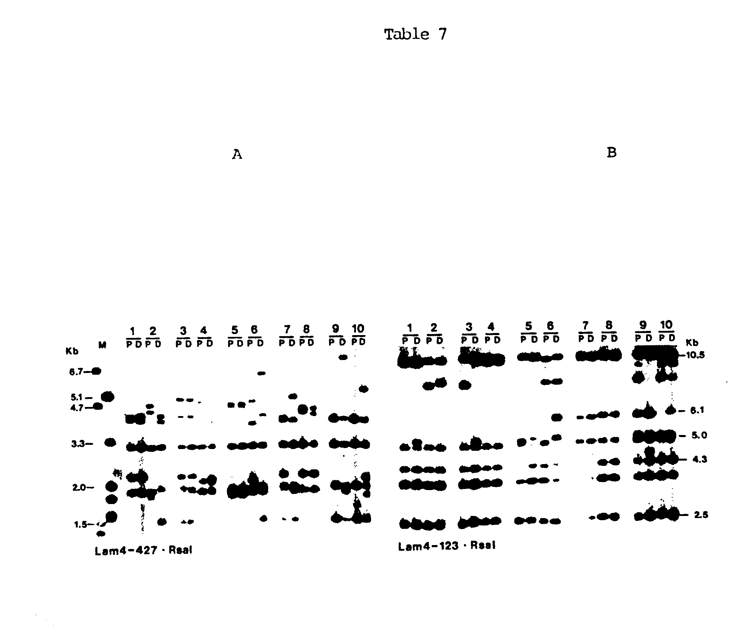

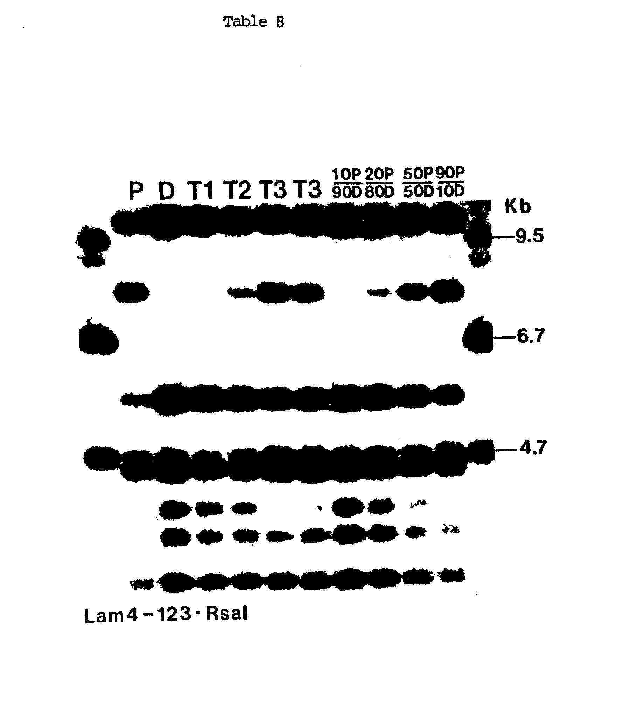

Table 3A. RFLP Probe Lam4-427 has a PIC of 0.95 with the single enzyme RsaI based on analysis of 15 grandparents from Utah kindred. The assignment of a single chromosomal locus for this probe was determined from inheritance studies on eight Utah families. This probe has a DNA rearrangement type polymorphism which is also demonstrated with TaqI. Digestions of the probe with EcoRI or HindIII separated the polymorphism into two distinct parts, each containing two bands in each individual studied. This it is known that the two largest bands of the TaqI polymorphism are from one end of the probe, and the next two are from the other end. The TaqI digest reveals the entire variability as does RsaI. Table 4A shows an autoradiograph of this probe hybridized to Rsa I cleaved DNA from 20 unrelated individuals (A-Q); molecular length markers are shown in kilobases to the left and right of the figure in Table 4A.

Table 3B. The pattern of hybridization of Lam4-1214 to BglII digests of 13 unrelated individuals (J-W) are shown. The cloned insert is 11Kb in length and contains no internal BglII sites; therefore, the cloned segment is derived from one of the BglII fragments larger than 11Kb. The PIC value 0.94 with the single enzyme BglII was estimated from analysis of 20 grandparental chromosomes from Utah kindred.

Table 3C. RFLP probe Lam4-123 demonstates at least 11 variable restriction fragment lengths in RsaI digests of DNA from random unrelated individuals. A PIC value of 0.86 was calculated from data obtained from 16 grandparents in Utah kindreds. Mendelian inheritance consistent with a single locus was confirmed by studies with 6 Utah families.

Table 3D. RFLP probe Lam4-355 displays a light constant band of 6.6Kb and at least 12 variable bands among BglII-digest DNAs from 14 unrelated individuals. Note the substantial diveristy of fragments present in the 3.1 to 5.2 Kb size range. The PIC value 0.90 was calculated from analysis of 28 chromosomes.

Table 3E. RFLP probe Lam4-159 hybridizes to a variable PstI fragment at one end of the probe and a different variable PstI fragment at the other end. Each of these length variations is also observed with other restriction enzymes indicating DNA rearrangement as the basis of the polymorphism. Different combinations of the two variable fragments per chromosome generate many allelic arrangements at this locus (12 alleles have been observed in 11 people of klnown genotype). The PIC value 0.90 was calculated from analysis of 30 chromosomes.

Table 3F.  RFLP probe Lam4-966 hybridizes to variable RsaI
fragments ranging in length from 2.3-5.3Kb.  Fourteen distinct
locus haplotypes have been identified in eight Utah families.  A
PIC value of 0.88 was calculated from the haplotype information
of 17 Utah kindred grandparents.

Table 3G.  RFLP probe Lam4-368 displays 6 variable fragments
(25, 20, 16, 11.5, 8.5 and 4.6Kb) and 8 constant fragments (9.6,
7.1, 4.5, 4.1, 3.9, 3.0., 2.9 and 2.4Kb) in this group of HindIII
digested DNAs from 10 unrelated individuals.  Note that each
individual displays only one or two variable fragments.  In fact,
for this probe-enzyme system, a single variable fragment size
defines each allele.

Table 3H.  RFLP probe Lam4-744 reveals a rearrangement type
polymorphism with the restriction enzyme TaqI, HaeIII, HindIII,
MspI and RsaI; RsaI demonstrates the largest number of variable
fragments with range in length from 1-4Kb.  A PIC value of 0.8
has been calculated using data derived from 20 random
individuals.

Table 3I.  RFLP probe Lam4-1065 reveals a rearrangement type
polymorphism with the restriction enzymes HindIII, MspI, TaqI and
RsaI.  A PIC value of 0.75 was calculated from the data obtained
from 17 grandparents from Utah kindred.  Twelve haplotyes have
been identified in eight Utah families studied.  At least 16
variable fragments ranging in length from about 1-8Kb have been
observed.

Table 3J.  RFLP probe Lam4-962 reveals 6 variable fragment
lengths with restriction enzyme MspI cleaved DNA.  A PIC value of
0.77 has been calculated from data obtained from 18 Utah kindred
grandparents.  Eight haplotypes have been identified in 9 Utah
families;

Table 4 contains examples of the Mendelian inheritance
of the variable fragments for each of the RFLP probes using
3 generation Utah families and the locus haplotypes derived
are shown.  These data were observed from analysis of the hybridization patterns of restriction enzyme cleaved DNAs purified from lymphoblastoid cell lines established from blood samples obtained from the Utah family members in gel transfer experiments;

Table 5 shows the genotyping strategy used in bone marrow transplant before transplant (pretransplant) and following transplant (post transplant);

Tables 6A - 6B show bone marrow transplant genotyping with a RFLP probe panel applied to 10 individual cases.

In Table 6A 10 individual cases probes, revealing patient and donor specific fragments, donor only, patient only or neither is tabulated for each of the RFLP probes constituting the panel summarized in Table 1. In all cases both donor and patient specific restriction fragments were detected with a single probe-enzyme combination.

In Table 6B 10 donor/patient pairs are illustrated the informativeness per probe is tabulated, e.g. RFLP probe Lam4-427 detected donor and patient-specific restriction fragments in 5 of the 10 cases, donor-specific fragments in 2 of 10 cases; therefore, in 7 of 10 cases the probes revealed donor or donor and patient specific fragments;

Table 7 contains a figure showing autoradiographs of the results of hybridization of the RFLP probes from the 10 donor/patient pairs numbered as in Table 6A and as described in Table 6A. As illustrated P = patient, D = donor;

Table 8 contains a figure showing lympho-hematopoietic chimerism following bone marrow transplantation. The reappearance of patient DNA following engraftment of donor hematopoietic cells is seen. P,D indicate pretransplant patient and donor RsaI digested DNAs hybridized with RFLP probe Lam4-123. T1 indicates DNA obtained from cultured peripheral blood drawn approximately 1 month post transplant; T2 indicates DNA purified from peripheral blood approximately 4 months after transplantation; T3, T3 indicates DNA purified from peripheral blood (left) or bone

marrow (right) approximately 5 months following transplantation. Four DNA admixture standards are included. 10P/90D indicates 10% patient DNA, 90% donor DNA (total sample = 4 micrograms). These DNAs were purified from blood samples obtained prior to transplantation. At four months following transplanation (T2) apparently 20% patient origin DNA was present, and at five months 75% patient origin DNA was present in both peripheral blood and bone marrow samples (T3). Proportions of DNA are estimated from a comparison of the signal intensity observed with the admixture DNA standards prepared under the same conditions.

## Description of the Preferred Embodiments

Phenotypically neutral DNA sequence variations occur among individuals of any given species and arise from the insertion or deletion of DNA segments or, more commonly, by single nucleotide base changes. Some sequence differences change restriction endonuclease recognition sites or their spacing. The sequence difference is then revealed as variation in the length of restriction fragments recognized by a cloned DNA probe when the DNA of two or more individuals is examined. In practice, total genomic DNA is cleaved with a restriction enzyme, separated according to size by electrophoresis, transferred and bound to a solid support such as a nylon membrane and hybridized to a radioactively labeled cloned single copy DNA probe. The restriction fragment length polymorphism (RFLP) is visualized by autoradiography as a difference in the pattern of bands produced by hybridization of the probe to genomic DNA.

Family studies have shown that the variations in fragment lengths, RFLPs, are inherited in a Mendelian fashion with one form or allele coming from each parent. Since the RFLPs are stably inherited they can be used as genetic markers useful in the construction of a human genetic linkage map as proposed by Botstein, et al. (1980) supra. Such RFLP probes reveal polymorphism at a single locus (e.g. a particular segment on a given chromosome). It has now been found that the sequence diversity among humans can also be used to distinguish among individuals in a much more sensitive way than any other currently practiced method of genotyping, e.g. HLA. However, all DNA polymorphisms are not equally useful. The most useful RFLPs from the standpoint of genetic mapping and diagnostic testing are those that exist in a large variety of fragment lengths among individuals in a given population and at a reasonable frequency for each of the alleles.

A measure of the quality of a RFLP is called its polymorphism information content (PIC) value. This is a mathematical expression (Botstein, et al. (1980) supra) which is a measure of heterozygosity. It is the probability that segregation and distribution of RFLP alleles can be traced in any given family. For example, a simple two allele system in which the frequency of each allele is 50% would give a PIC value of 0.37. Because the PIC is based on the number and frequency of RFLP alleles in the population, it can also be used as a measure of the likelihood that two unrelated individuals will have different restriction fragment lengths with a RFLP probe. So, in order to have a high probability of distinguishing the genotype of two unrelated individuals, RFLP probes with high PIC values (in practice $\geq$ 0.7 PIC) are preferred for use.

It has been estimated that approximately 70% of the human genome is comprised of unique i.e. single-copy DNA sequence and 20% mid repeat sequences (approximate repeat 2-1000 times per genome) while about 10% is highly repeated 105 fold repeat sequences (Strayer, D., Heintz, N., Roeeler, R. and Gillespie, D. (1983) Proc. Natl. Acad. Sci. USA 80: 4770-4774). While polymorphism exists in all three classes of human sequence, variation between individuals at single-copy sequence regions is most useful for the genotyping tests described in this invention. Only unique sequence regions are tested for RFLPs by hybridization of cloned single-copy DNA sequences to restriction endonuclease cleaved DNAs from unrelated individuals in gel-transfer experiments (Wyman and White (1980) supra and White, et al.(1985) supra).

The ten useful probes of this invention were obtained by suitable selection from a human genomic library. The following discussion indicates how such probes can be obtained.

Human genomic libraries have been constructed which contain cloned DNA segments 10-20 Kilobases in length (Lawn, et al.(1978) supra). Putative single copy clones are identified from plaque filter hybridization experiments in which total genomic human DNA is labeled with P32 via a nick translation reaction (Rigby, P.W.J., Dieckmann, M., Rhodes, C. and Berg, P. (1977) J. Mol. Biol. 113: 237-251) and hybridized to filter replicas of bacteriophage clones containing human DNA inserts plated on a lawn of E. coli (Wyman and White (1980) supra). Bacteriophage plaques that show no hybridization as judged from the autoradiograph of the nitrocellulose filter are candidate single-copy DNA containing clones. Clones which show strong hybridization signals contain middle repetitive to highly repetitive human DNA sequences; these are avoided in favor of those that contain human DNA but do not hybridize to the total human DNA probe.

Each candidate single copy human DNA clone can be tested for the ability to reveal polymorphism by hybridization to restriction enzyme cleaved genomic DNA from random unrelated individuals. The bacteriophage clones containing the human DNA inserts are radioactively labeled with P32 via nick translation and hybridized in gel transfer experiments to genomic DNAs. A standard screening blot consisting of 5 unrelated individuals cleaved with each of 6 restriction enzymes (e.g. MspI, TaqI, HindIII, EcoRI, BglII, BamHI) is efficient in detecting polymorphism. Since ten chromosomes scored a two allele polymorphism (frequency of each allele 50%) or greater than 2 allele polymorphism will be detected, if present, with a certainty of greater than 90% using such a screening panel.

Once polymorphism has been detected follow up gel transfer experiments are done with panels of DNA (cleaved with the appropriate restriction enzyme) from more random unrelated individuals (e.g. 20) so that an estimate of the frequency of the RFLP alleles can be made and PIC can be estimated. In addition, other enzymes can be tested to see if additional variation can be detected. It is important to confirm that the polymorphism represents a single locus. This is done by studying the inheritance of the RFLP alleles in families. The presence of more than one locus would be suspected if non-Mendelian segregation of the banding pattern in the autoradiograms is observed. In some cases the polymorphic patterns are very complicated and alleles can only be defined from such inheritance studies. Useful family pedigrees for these analyses contain the 4 grandparents, the parents, and large sibships (6 or more children) from which DNA can be purified from blood samples obtained or isolated from immortalized lymphoblastoid cell lines (Cambden Cell Repository, White, et al. (1985) supra).

Ten RFLP probes with PIC values $\geq 0.7$ have now been identified which can be used for genotyping purposes (found as defined in Table 1). Each of the RFLP probes was identified from screening random single-copy clones from a human genomic DNA library (10-20 Kb human DNA inserts cloned into the bacteriophage lambda vector Charon 4A (Lawn, R.W., Fritsch, E.F., Parker, R.C., Blake, G and Maniatis, T. (1978) Cell 15: 1157-1174, and Wyman and White (1980 supra)). Each probe detects polymorphism completely independent of the others. None of the polymorphic moci are genetically linked, i.e. they are situated on different

chromosomes or are more than 40 centimorgans (40% recombination fraction) from each other, as determined by testing co-inheritance in families as described by White, et al. (1985) supra. As described in this invention, the set of probes is ideally suited for discrimination of individual genotypes.

In exclusion of paternity, for example, it is necessary to show that a RFLP allele inherited by the child is not carried by the putative father. If this man is not the father and is unrelated to the true biological father, then the probability of failing to find such an allele with a polymorphic probe is approximately (1 - PIC). If a series of independent RFLP markers are tested, the probability that a random person would contribute the same alleles as the true father decreases to

$$\prod_{i=1}^{N} (1 - PIC_i)$$

where N is the toal number of probes used and $PIC_i$ is the polymorphism information content of the ith marker. This value for the set of markers described here is:

(0.05) (0.06) (0.10) (0.10) (0.14) (0.12) (0.2) (0.2)
(0.25) (0.23) = 1.1x10-9

The likelihood of excluding paternity by a person not related to the true father is therefore far greater than 99.99%.

In bone marrow transplantation (and in some paternity cases) the individuals to be compared are related, and use of a single RFLP probe (no matter how high the PIC) cannot guarantee that their alleles can be distinguished. Two siblings, for example, have a 25% chance of inheriting the same two alleles from the parents they share, and an additional 50% chance of having at least one allele in common. However, even with this limitation, the likelihood of distinguishing sibling genotypes with a series of probes with PIC 0.7 is very high. The probability that two siblings are indistinguishable at a single polymorphic locus is approximately

$$\frac{1}{4} + \frac{1}{2} \sum_{i=1}^{n} f_i^2$$

where $f_i$ is the frequency in the population of allele i and n is the total number of alleles at the locus. The probability of not detecting an allele difference with any of the set of probes described in this invention is then the product of this probability for each individual probe; for these 10 probes, this value is 1.3 x 105. Application of this set of probes to analysis of bone marrow transplants will be useful in more than 99.99% of cases.

It must be noted that use of additional available RFLP markers can improve the numbers further. It should be noted that not all genotypic differences are equally detectable in practice, so that the numbers will be somewhat less favourable than theoretically predicted. It is for this reason that a conservative number of 99.99% was chosen as the likelihood of detecting non paternity or distinguishing siblings in bone marrow transplants.

The properties of the ten RFLP DNA probes of this invention are summarized in Table 1. Restriction maps of the ten RFLP DNA probes are shown in Table 2. Example autoradiographs of RFLP probes hybridized to restriction enzyme digests of DNA from unrelated individuals demonstrating the variable fragment lengths are shown in Table 3A - 3J. Additional notes on each of the probes are given in the section of brief description of the drawings.

The general procedure for carrying out an identity test using RFLP DNA probes is composed of three main steps. The first step involves the isolation of human genomic DNA. The human genomic DNA is prepared from any source as for example from either an effective amount of peripheral blood, cultured cells or bone marrow aspirates. The cells are lysed by the addition of buffer. Preferably a ten fold volume of buffer is used but the volume can vary. The buffer is preferably 0.32M sucrose, 10 mM Tris-HCl (pH 7.5), 5 mM $MgCl_2$ and 1% Triton-X-100 but concentrations of the buffer components can vary widely as can the lysing buffer itself since any buffer found capable of lysing such cells can be used. The nuclei are preferably isolated by centrifugation and suspended in a small volume of buffer. The buffer preferably contains 75 mM NaCl, 25 mM EDTA (ethylene diamine tetraacetic acid) and 1% SDS (sodium dodecyl sulfate) but the actual components can vary widely. To the suspended nuclei is added proteinase K preferably at a concentration of 10 mg/ml and incubated overnight at $42^{o}$C. The incubation period is preferably from 12 to 16 hours but this period can be altered depending upon the amount of proteinase K used and the temperature of incubation. Following extraction of the solution preferably at room temperature for 2-4 hours with Tris-EDTA saturated phenol, the aqueous phase is removed with a solution of chloroform and isoamylalcohol (preferably 24:1) and the DNA precipitated preferably by the addition of one tenth volume of 3 M NaCl and 1.5 volume isopropanol or a similar DNA precipitating solution. The DNA is dissolved preferably in TE buffer (1 mM Tris-HCl (pH 7.5) containing 1 mM EDTA) and reprecipitated by addition of 0.5 volume 7.5 M ammonium acetate and 2 volumes of cold 95% ethanol. The DNA is resuspended preferably in 10 mM Tris-HCl (pH 7.5) containing 1 mM EDTA to a concentration of preferably 250 micrograms/ml and stored at $4^{o}$C. The DNA yield is approximately 100-200 micrograms per 10 ml peripheral blood or 10 micrograms per 106 cells.

In the second step the isolated DNA is digested with a restriction enzyme. DNA samples at a final concentration of preferably 175 micrograms/ml are digested to completion with restriction endonuclease under conditions recommended by the supplier. The digestion period is preferably 4 to

6 hours but the digestion period can vary. The amount of restriction endonuclease used to digest the DNA is preferably 5 units per microgram but the amount of restriction endonuclease can vary a great deal dependent in part upon the particular restriction endonuclease and the digestion conditions employed. The completeness of digestion is monitored by removing aliquots of the reaction mixture preferably containing about 1 microgram human genomic DNA and adding to an identical reaction mixture containing preferably 1 microgram of bacteriophage lambda DNA or another DNA known to be digested by the restriction endonuclease. The amount of both DNAs can vary but the amounts of both DNAs in each variation is the same. A limit digest of the genomic DNA is assumed if the digest pattern of lambda DNA showed complete cleavage as measured by ethidium bromide staining of the DNA size-fractionated by electrophoresis in an agarose gel preferably 0.8%.

Thirdly, the resulting restriction endonuclease cleaved DNA is size-fractionated by electrophoresis. The electrophoresis is preferably carried out through a 0.8% agarose gel submerged in Tris-acetate-EDTA buffer. The percent agarose gel and the buffer components may vary greatly. Following electrophoresis, the DNA is denatured, neutralized and transferred to a membrane. The DNA is preferably denatured in the gel with a solution of 0.2N NaOH and 0.6M NaCl, neutralised with 0.5 M Tris-HCl (pH 8.0) and 1.5 M NaCl, and transferred to a membrane in 25 mM sodium phosphate (pH 6.5). The buffer components may vary as known in the art as long as the treatment of the DNA as desired is accomplished. The membrane is preferably nylon as supplied as zeta-bind of AMF Cuno and the transfer time is preferably 12 to 16 hours but the time can vary from as little as 30 minutes or less or to 48 hours or more. Following transfer, the membrane is washed and then baked preferably for 2 hours in a vacuum oven at $80^{\circ}$C. The DNA containing membrane is then prehybridized from 2 hours or less to 24 hours or more at preferably $42^{\circ}$C. The prehybridization solution is preferably 5x SSC, 40 mM sodium phosphate (pH 6.5), 0.1 mg/ml

-18-

salmon sperm DNA, 10% dextran sulfate, 50% formamide, 5x Denhardts (Ficoll 400, polyvinyl pyrrolidone 360, bovine serum albumin). RFLP DNA probes (as bacteriophage lambda clones, human DNA inserts preferably 10-20Kb) are labelled with 32P to a specific activity preferably greater than 108 dpm/microgram via a nick translation reaction with DNA polymerase I and alpha-32PdNTPs. The labelled DNA is separated from unincorporated nucleotides either by spermidine precipitation or by chromatography on a support such as Biogel P-60 (Rio-Rad). The 32P labelled DNA is denatured preferably by boiling for five minutes then added to the prehybridization solution containing the nylon membrane. Hybridization is carried out preferably at $42^{O}$C. for 20 hours. Following hybridization, membranes are washed preferably for 30 minutes at room temperature with 2x SSC and 60 minutes at $65^{O}$C. in 0.1xSSC containing 0.2% SDS. Hybridization results in DNA banding patterns which are visualized following autoradiography. The various buffer components and conditions of the hybridization procedure may vary as known in the art as long as the desired DNA patterns are obtained.

Besides radioactive labelling, DNA probes can be labelled by any of a variety of known labelling techniques including incorporating protein or biotin coupled nucleotides. Protein or biotin coupled nucleotides, once incorporated into the probe, can also visualize DNA patterns through the use of fluorescent antibodies binding to the protein or biotin or to non-fluorescent antibodies also bound to such molecules. These complexes can then be visualized by illumination with ultraviolet light.

Application to Bone Marrow Transplantation Studies

This test uses RFLPs to follow the genetic lineage of any nucleated cells which arise in the bone marrow of patients who have undergone bone marrow transplantation. This allows the distinction of the donor's cells from those of the recipient on the basis of their DNA. The test requires only a small amount of DNA and a set of probes which reveal polymorphism in substantially every case. In addition, the test is designed to allow detection and study of patients with mixed hematopoietic cell populations following transplantation. In most cases detection of 10% admixtures and in some cases as little as 1% admixture can be detected.

The object of the genotyping, in this case, is to identify donor-specific and patient-specific cells by their restriction fragments that are revealed by the RFLP probes so that the presence of either or both types of cells in the patient's hematopoietic cells following transplantation can be detected. Important considerations are that the fragments be clearly separated from one another and give a strong hybridization signal so that ambiguities are minimized. It is also important that the test be possible on small quantities of DNA since this is often a limiting quantity in patients following transplantation, especially in children undergoing transplantation. Finally the test allows the detection of a small fraction (about 5% or less) of one cell type in the presence of 95% of the other.

The analysis proceeds in two phases as diagrammatically illustrated in Table 5; first a pretransplant genotyping screen is done for donor and patient. The results of the analysis allow selection of a RFLP probe which is used to study the DNA of the patient in any subsequent tests after transplantation has taken place. As calculated above, it is a certainty to 99.99% that such a probe can be found among the 10 listed (Table 1).

In the first phase prior to transplantation, blood samples (10-20 ml) are collected from the donor and patient and the DNA purified.

A panel of RFLP probes is then tested against the donor and patient DNAs in gel-transfer hybridization experiments in order to find donor and patient specific patterns. In order to be efficient in the process RFLP probes can be applied sequentially beginning with the top set of 4 RFLP probes (see Table 1). PICs of these probes range from C.86-0.95. Each of these probes requires either restriction endonucleases RsaI or BglII to reveal the polymorphism. Thus only two digests are required and, since the nylon membranes containing the donor and patient DNAs can be reused, the membranes can be reprobed sequentially. Aliquots of donor and patient DNA are cleaved with the appropriate restriction endonuclease, fractionated according to size by electrophoresis through an 0.8% agarose gel, transferred to a solid support (nylon membrane) and hybridized with RFLP probes.

For example, in a typical case RsaI and BglII digests are done; DNA is transferred to a nylon membrane and hybridized with the top 4 RFLP probes, e.g. Lam4-427 hybridized to a membrane containing donor and patient DNA cleaved with RsaI or BglII. The RFLP patterns visualized by autoradiography are analyzed and the best RFLP probe/enzyme combination (i.e. one which shows one or more clearly identifiable donor-specific and patient-specific restriction fragments that are well separated and give strong hybridization signals) is chosen for use in follow up studies after transplantation. If both criteria are not satisfied by the 1st probe screen, a second set of probes are tested, e.g. the next 6 RFLP probes on the list with samples digested with additional enzymes as needed until donor specific and patient specific patterns are found. The most desirable situation is one in which a single probe/enzyme combination detects donor and patient specific fragments; in some cases it may be necessary to use two RFLP probes.

In the second phase after transplantation, the progress of engraftment is monitored by analysis of DNA purified from blood samples or less typically, from bone marrow. For example, DNA purified from a blood sample drawn 30 days post transplant is cleaved with the restriction enzyme appropriate for the RFLP probe chosen from the pretransplant analysis, and nylon membranes (blots) prepared containing pretransplant donor and patient DNA cleaved with the same enzyme in addition to the post transplant sample. Internal control DNA standards are also prepared by digesting admixtures of pretransplant donor and patient DNAs with the same enzyme selected from the genotyping studies. Various percentages of donor and patient DNAs are mixed e.g. 10% patient + 90% donor, 50% of each, 90% patient + 10% donor. If mixed lymphohematopoietic chimerism is detected from such a post transplant study, additional follow up studies with DNAs isolated from cell fractions may be performed to determine patient or donor origin of each cell type (T cells, B cells, monocytes etc.).

With this RFLP probe panel it is possible to distinguish virtually any two individuals, even siblings, with a probability of success greater than 99.9%. (Of course identical twins are excluded since they share the same genetic material).

Application to Paternity Testing

In this case the objective is to determine exclusion of paternity values for any given putative father. A set of RFLP probes is applied to DNA obtained from the mother, child and putative father. RFLP patterns are compared and the bands potentially contributed by the mother to the child are identified for each probe used. The alleles that must have been contributed by the father can then be identified by subtraction and compared to the RFLP alleles of the putative father. If the putative father does not carry the child's paternal alleles, non paternity is proved. From patterns seen between the child and the putative father a calculation of exclusion probability can be made. As shown above exclusion of paternity to a level of 99.99% can be achieved with the set of RFLP probes described (Table 1).

Blood samples (1-20 ml) are collected from the mother, child and putative father and DNA purified. The three DNAs are digested with the enzymes required as listed in the

probe panel (Table 1), size fractionated on a 0.8% agarose gel and transfered to a nylon membrane. The 10 DNA RFLP probes are labeled with P32 and hybridized separately to the enzyme cleaved DNAs from the three individuals. Results are interpreted from autoradiographs of the hybridized membranes. Any non-identities found in the putative father's patterns (alleles) that remain after the mother's contribution has been subtracted count as exclusion. The exclusion probability is then calculated as indicated above.

Example 1: Genotyping 10 donor/patient pairs using the RFLP probe set of Table 1 to determine which probes of the set can be used in a later test to identify DNA of the patient and of the donor.

In order to demonstrate the ability of the RFLP probes to reveal sufficient polymorphism such that donor specific and patient specific patterns could be detected in bone marrow transplant patients and followed after transplantation in the patient, DNA from 10 donor/patient sibling pairs was tested against the probe set Table 1. In all cases, donor specific and patient specific restriction fragments were detected for each pair with at least one probe. The data is summarized in Table 6.

Two micogram aliquots of human genomic DNA were digested with the enzymes MspI, TaqI, RsaI, BglII, HindIII, and PstI and size fractionated by electrophoresis through an 0.8% agarose gel. The DNA was denatured, transferred to nylon membranes and hybridized with P32 labeled RFLP probes. Complete digestion was monitored by digestion of a 1 microgram sample of genomic DNA from the original digest with 1 microgram sample of genomic DNA from the original digest with 1 microgram of bacteriophage lambda DNA; complete digestion was assumed from the characteristic limit digest pattern of the lambda DNA visualized by ethidium bromide staining of the size fractionated DNA. The results from the autogradiographs were tabulated according to whether a RFLP probe revealed donor specific, patient specific or both types of restriction fragments (see Table 6).  Example autoradiographs showing the results of hybridization of RFLP probes Lam4-427 and Lam 4-123 to RsaI cleaved DNA from the 10 donor/patient pairs is shown in Table 7 (A and B).  Tables 6 and 7 show, for example, that probes 1214, 159 and 355 are informative for both patient and donor pair numbered 1; probes 123 and 962 are informative for the donor of pair numbered 1 only and are probes 427, 966, 368, 744 and 1065 are not informative for the donor or patient of pair 1.

Example 2:   Documentation of Lympho-Hematopoietic Chimerism in a Patient Following Bone Marrow Transplantation

Engraftment of donor marrow is followed by reappearance of cells of patient origin as shown by the autoradiograph given in Table 8.  Genomic DNA was cleaved with restriction endonuclease RsaI, size fractionated by electrophoresis through a 0.8% agarose gel and transferred to a nylon membrane.  Hybridization with RFLP probe Lam4-123 (previously identified with pretransplant donor/patient DNA samples as detecting donor and patient specific RFLP patterns) labeled with P32 demonstrates the presence of both donor origin and patient origin hematopoietic cells in the patient following transplantation.  T1-T3 indicate patient samples drawn 1-5 months following transplantation and were either DNA purified from peripheral blood or cultured peripheral blood samples.  T3 (far right) represents DNA purified from bone marrow aspirates.  Lanes indicated P or D are pretransplant DNA samples digested under the same conditions as the post transplant samples (T1-3).  Admixtures of pretransplant donor and patient DNAs were also digested with RsaI and electrophoresed in parallel with the other samples.  The relative proportions of donor or patient DNA in the total 2 microgram sample are indicated e.g. 10P/90D indicates 10% patient DNa and 90% donor DNA.

Example 3:   Parentage Testing:   Exclusion of Paternity

Two micrograms of genomic DNA purified from blood samples obtained from the mother's child and putative father are digested with restriction enzyme and hybridized with RFLP probe Lam4-1214 in a gel transfer experiment. The restriction fragments observed from the child's DNA are consistent with inheritance of RFLP alleles from the mother and putative father. Fragments present in the putative father's restriction enzyme digest and not present in the mother's DNA are apparent in the DNA of the child. Exclusion of paternity is virtually ruled out by comparison of the RFLP patterns of the mother, child and putative father. 10 RFLP probes sufficiently polymorphic to exclude paternity with 99.99% probability are used in the analysis. In every case the genotypic patterns in gel-transfer (Southern blot) hybridizations are consistent with the expected paternity. A priori, it is expected from the PIC's of the probes used that the probability of non-paternity (i.e., the probability that the same alleles would be contributed by a random person) would be $1.4 \times 10-12$. (For a highly polymorphic marker, the probability of two individuals contributing the same allele is very close to (1-PIC)).

In practice, confidence is significantly lower because one cannot unambiguously determine maternal and paternal alleles from the band patterns of these three family members. However, on the basis of the frequency of alleles with particular bands inherited by the child from the father, one can calculate that the probability of non-paternity is $2.8 \times 10-8$.

While specific examples of this invention have been shown and described, many modifications can be made as will be recognized by those skilled in the art. For example, the bone marrow and genotypic tests of this invention while illustrated on humans, can find application in a number of animals after selection of suitable sets of probes applicable to the animal species involved.

## Claims

1. A method for genotypic testing based on DNA restriction fragment length polymorphism, said method comprising:

digesting first and second DNA from a first individual and a second individual respectively with a restriction endonuclease that produces a polymorphic digestion pattern in the first and second DNA;

subjecting the digested first and second DNA to hybridization using a set of labeled RFLP DNA probes which set of probes contain at least one appropriate probe to distinguish said first individual from second individual; and

comparing DNA patterns from the first individual and second individual to distinguish the DNA origin with high accuracy.

2. A method according to Claim 1, wherein said probe is from the group consisting of Lam4-427, Lam4-1214, Lam4-123, Lam4-355, Lam4-159, Lam4-966, Lam4-368, Lam4-744, Lam4-1065 and Lam4-962.

3. A method according to Claim 1, and further comprising the step of testing DNA from the bone marrow of one of said individuals by hybridizing with said DNA at least one probe to determine if said last mentioned DNA matches first or second DNA.

4. A method according to Claim 3, wherein said probe is from the group consisting of Lam4-427, Lam4-1214, Lam4-123, Lam4-355, Lam4-159, Lam4-966, Lam4-368, Lam4-744, Lam4-1065 and Lam4-962.

5. A method according to Claim 1, and further comprising testing a third individual to determine relationship to said first and second individual, said last mentioned testing comprising, hybridizing DNA from said third individual with said at least one appropriate probe and comparing patterns of said last mentioned DNA with patterns of said first and second DNA to determine relationship.

6. A method according to Claim 5, wherein said probe is from the group consisting of Lam4-427, Lam4-1214, Lam4-123, Lam4-355, Lam4-159, Lam4-966, Lam4-368, Lam4-744, Lam4-1065 and Lam4-962.

7. A method according to Claim 1, wherein said first and second DNA is isolated from peripheral blood.

8. A method according to Claim 1, wherein said first and second DNA is isolated from bone marrow fluid.

9. A method according to Claim 1, wherein said probes are labelled with a radioative nucleotide.

10. A probe selected from the group consisting of Lam4-427, Lam4-1214, Lam4-123, Lam4-355, Lam4-159, Lam4-966, Lam4-368, Lam4-744, Lam4-1065 and Lam4-962.

11. The probe of Claim 10, wherein the probe is Lam4-427.

12. The probe of Claim 10, wherein the probe is Lam4-1214.

13. The probe of Claim 10, wherein the probe is Lam4-123.

14. The probe of Claim 10, wherein the probe is Lam4-355.

15. The probe of Claim 10, wherein the probe is Lam4-159.

16. The probe of Claim 10, wherein the probe is Lam4-966.

17. The probe of Claim 10, wherein the probe is Lam4-368.

18. The probe of Claim 10, wherein the probe is Lam4-744.

19. The probe of Claim 10, wherein the probe is Lam4-1065.

20. The probe of Claim 10, wherein the probe is Lam4-962.

21. A method in accordance with Claim 1, wherein said set of probes comprise at least two probes having a PIC value of greater than 0.7.

22. A method according to Claim 21, wherein said probe is from the group consisting of Lam4-427, Lam4-1214, Lam4-123, Lam4-355, Lam4-159, Lam4-966, Lam4-369, Lam4-744, Lam4-1065 and Lam4-962.

Table 1

RFLP Probe Panel

| Probe | Enzyme | PIC | Rearrangement or Site Polymorphism | Length (Kb) Human Insert | Locus Haplotype (K1331)(Kb) | Constant Fragments (Kb) | Add'l Polymorphism Related | Unrelated |
|---|---|---|---|---|---|---|---|---|
| Lam4-427 | RsaI | 0.95 | R | 17.0 | (1) 4.3,2.1,2.0<br>(2) 4.4,2.1,2.0<br>(3) 4.1,2.4,2.1<br>(4) 4.7,2.1,2.0<br>(5) 4.1,2.3,2.1<br>(6) 4.3,2.1,2.05<br>(7) 4.2,2.4,2.1<br>(8) 4.0,2.4,2.1 | 3.2 | E,M,H,T | — |
| Lam4-1214 | BglII | 0.94 | R | 11.0 | (1) 12.0,9.5,8.2<br>(2) 12.0,9.5,5.6<br>(3) 11.5,5.6,5.2<br>(4) 12.0,9.5,4.3 | — | B,H,E,Bc,Pv,X | — |
| Lam4-123 | RsaI | 0.86 | R | 14.5 | (1) 10.0,4.3,3.5<br>(2) 5.5,4.5,10.0<br>(3) 7.7,4.3,3.5 | 3.0,1.2,1.0 | M,T | H,Bg,Hc |
| Lam4-355 | BglII | 0.90 | R | 12.0 | (1) 6.6,3.5<br>(2) 6.6,10.2,3.3<br>(3) 6.6,3.8,3.5<br>(4) 6.6,10.2,3.7<br>(5) 6.6,10.2,3.5 | 1.3 | B,Ha,H,M | — |
| Lam4-159 | PstI | 0.90 | R | 13.6 | (1) 6.0,5.4,1.9<br>(2) 6.0,4.2,2.35<br>(3) 6.0,4.7,2.3<br>(4) 6.0,4.6,2.3<br>(5) 6.0,4.7,2.25<br>(6) 6.0,4.2,1.95<br>(7) 6.0,4.8,2.3 | 6.4,2.2 | T,M,Pv,B | — |

Table 1. (continued)

| Probe | Enzyme | PIC | Rearrangement or Site Polymorphism | Length (Kb) Human Insert | Locus Haplotypes (K1331) (Kb) | Constant Fragments (Kb) | Add'l Polymorphism Related | Unrelated |
|---|---|---|---|---|---|---|---|---|
| Lam4-966 | RsaI | 0.8.8 | S | 12.2 | (1) 5.1<br>(2) 3.7<br>(3) 3.3<br>(4) 3.0<br>(5) 2.6 | 2.0,1.4,1.1,0.9 | -- | H |
| Lam4-368 | HindIII | 0.80 | S | 16.0 | (1) 8.5<br>(2) 11.5<br>(3) 4.5 | 9.6,7.4,4.5,4.1<br>3.9,3.1,2.8,2.4 | — | T,M,E,R |
| Lam4-744 | RsaI | 0.80 | R | 13.0 | Not. Det. | 4.0,1.6,1.2,0.8 | H,Ha,M,T | -- |
| Lam4-1065 | RsaI | 0.75 | R | 12.0 | (1) 5.4,0.9<br>(2) 4.0<br>(3) 3.7<br>(4) 3.2<br>(5) 2.7 | 1.9,1.5,0.8 | H,Ha,M,T | — |
| Lam4-962 | MspI | 0.77 | S | 15.9 | (1) 3.7,2.0,1.7<br>(2) 4.3,2.3,1.7<br>(3) 3.7,2.3,1.8 | 3.5,3.0,1.0 | -- | -- |

Table 2A

Lam4-159

| | | | | | | |
|---|---|---|---|---|---|---|
| BamHI | 0 | 5.6 | 38.7 | 39.3 | 43.0 | 44.5 |
| HindIII | 0 | 23.3 | 24.1 | 27.3 | 38.8 | 44.5 |
| EcoRI | 0 | 19.9 | 27.9 | 31.7 | 33.5 | 44.5 |

Lam4-1214

| | | | | | | | | | |
|---|---|---|---|---|---|---|---|---|---|
| BamHI | 0 | 5.6 | 20.5 | 21.3 | 22.1 | 23.9 | 25.7 | 26.9 | 28.1 | 29.5 |
| | | 35.1 | 35.5 | 39.4 | 40.8 | | | | |
| BglII | 0 | 0.5 | 31.3 | 31.7 | 32.3 | 32.4 | 37.2 | 39.6 | 40.8 |
| HindIII | 0 | 35.3 | 40.8 | | | | | | |
| EcoRI | 0 | 19.9 | 30.9 | 40.8 | | | | | |

Lam4-744

| | | | | | | |
|---|---|---|---|---|---|---|
| BamHI | 0 | 5.6 | 23.0 | 38.0 | 38.5 | 42.3 | 43.8 |
| HindIII | 0 | 25.8 | 38.2 | 43.8 | | | |
| EcoRI | 0 | 19.9 | 32.9 | 43.8 | | | |

Lam4-966

| | | | | | | | | | |
|---|---|---|---|---|---|---|---|---|---|
| BamHI | 0 | 5.6 | 20.6 | 24.6 | 27.0 | 28.9 | 37.1 | 37.7 | 41.6 | 43.1 |
| HindIII | 0 | 25.6 | 29.6 | 31.1 | 37.6 | 43.1 | | | |
| EcoRI | 0 | 19.9 | 26.1 | 32.1 | 43.1 | | | | |

Lam4-1065

| | | | | | | | | | |
|---|---|---|---|---|---|---|---|---|---|
| BamHI | 0 | 5.6 | 27.2 | 37.1 | 37.6 | 41.5 | 43.0 | | |
| HindIII | 0 | 24.1 | 25.3 | 37.3 | 41.5 | 43.0 | | | |
| EcoRI | 0 | 19.9 | 20.6 | 27.7 | 28.9 | 29.9 | 30.7 | 32.1 | 43.0 |

Lam4-123

| | | | | | | |
|---|---|---|---|---|---|---|
| BamHI | 0 | 5.6 | 28.5 | 39.5 | 40.0 | 43.9 | 45.5 |
| HindIII | 0 | 21.8 | 26.2 | 39.8 | 45.5 | | |
| EcoRI | 0 | 19.9 | 23.4 | 29.0 | 34.4 | 45.5 | |

Lam4-962

| | | | | | | | | | |
|---|---|---|---|---|---|---|---|---|---|
| BamHI | 0 | 5.6 | 23.6 | 28.8 | 33.1 | 33.8 | 40.8 | 41.2 | 45.1 | 46.8 |
| HindIII | 0 | 20.8 | 21.8 | 26.9 | 33.0 | 41.0 | 46.8 | | |
| EcoRI | 0 | 19.9 | 28.2 | 32.8 | 35.8 | 46.8 | | | |

TABLE 2A Restriction Map

R = Eco RI    H = Hind III    B = Bam HI    K = Kpn I    Bg = Bgl II

0221633

Table 2B

Lam4-355

| BamHI | 0 | 5.6 | 27.4 | 29.2 | 29.9 | 30.7 | 37.0 | 37.5 | 41.4 | 42.9 |
| EcoRI | 0 | 19.9 | 26.1 | (28.9 or 29.3) | 31.9 | 42.9 | | | | |
| HindIII | 0 | 20.0 | 21.2 | 24.1 | 27.4 | 37.2 | 42.9 | | | |

Lam4-368

| EcoRI | 0 | 19.9 | 35.2 | 46.2 | | |
| HindIII | 0 | 26.3 | 30.5 | 32.9 | 40.5 | 46.2 |

Lam4-427

| BamHI | 0 | 5.6 | 22.5 | 42.1 | 42.6 | 46.5 | 48.0 | | | |
| BglII | 0 | 0.5 | 22.4 | 36.7 | 38.6 | 39.0 | 39.6 | 39.7 | 44.5 | 46.7 | 48.0 |
| KpnI | 0 | 17.4 | 19.0 | 25.3 | 44.5 | 48.0 | | | | |

## TABLE 2B  Restriction Map

Table 3A

Lam4 – 427 · RsaI

PIC 0.95

Table 3B

_Bgl II_

J K L M N O P Q R S T U V W

| Kb |
| --- |
| 25.0 |
| 15.0 |
| 12.0 |
| 9.5 |
| 6.6 |
| 4.3 |

9/42

0221633

Table 3C

Kb
— 9.5
— 6.7
— 4.7
— 1.6

0221633

Table 3D

Table 3E

A B C D I G H J K L O R S U V W Q B(A)

— 6.7

— 4.7

— 1.6

0221633

Table 3F

0221633

Table 3G

0221633

Table 3H

Table 3I

23.7

12.4
11.7

9.5

6.7

4.7

1.6
1.5

.8

Table 3J                                    0221633

Table 4A

# TABLE 4A

PROBE: $\lambda$ 4.427   ENZYME: R   KINDRED # 1345   PERSON/DATE:

---

Description of new locus haplotypes for this probe-enzyme

Constant fragments (if not already described):

Variable fragments for each new haplotype:

| Haplotype # 22 = 4.1, 2.3, 2.1 |
| Haplotype # 23 = 4.7, 2.1, 2.0 |
| Haplotype # 24 = 6.4, 2.4 |
| Haplotype # 25 = 4.0, 2.8 |
| Haplotype # 26 = 4.0, 2.1 |
| Haplotype # 27 = 4.0, 2.3, 2.1 |
| Haplotype # 7 = 4.2, 2.4, 2.1 |
| Haplotype # 11 = 4.1, 2.2, 1.0 |

Comments:

Table 4B

# TABLE 4B

PROBE: LAM4-1214  ENZYME: Bg   KINDRED #1345   PERSON/DATE:

Description of new locus haplotypes for this probe-enzyme
Constant fragments (if not already described):

Variable fragments for each new
haplotype:

| Haplotype# | | | |
|---|---|---|---|
| Haplotype# 15 | 23 | 9.5 | 8.2 |
| Haplotype# 25 | 20 | 6.3 | 5.2 |
| Haplotype # | | | |
| Haplotype # 1 | 12 | 9.5 | 8.2 |
| Haplotype# 9 | 20 | 5.6 | 5.2 |
| Haplotype# 10 | 12 | 5.6 | 5.2 |
| Haplotype# 17 | 12 | 9.3 | 7.4 |
| Haplotype# | | | |

Comments:
1345-10 also has 5.2 kb band.

Table 4C

# TABLE 4C

PROBE: λ 4-123     ENZYME: R     KINDRED #12     PERSON/DATE:

Description of new locus haplotypes for this probe-enzyme

Constant fragments (if not already described):

Variable fragments for each new haplotype:

| Haplotype | | |
|---|---|---|
| Haplotype# 1 = 10 | | |
| Haplotype # 2 = 7.7 | | |
| Haplotype # 3 = 9.8  5.5  3.9 | | |
| Haplotype # | | |
| Haplotype # | | |
| Haplotype# | | |
| Haplotype # | | |
| Haplotype# | | |

Comments:

0221633

Table 4D

# TABLE 4D

PROBE: λ4-355  ENZYME: Bg  KINDRED #1345  PERSON/DATE:

```
  (17,18)──[19,19]        (15,20)──[18,14]
    13       12             11       10

        (18,19)──────────[20,18]
          02                01
```

Offspring:

| 19,20 | 18,20 | 18,18 | 18,18 | 19,20 | 18,20 | 19,20 |
|-------|-------|-------|-------|-------|-------|-------|
| F     | M     | M     | M     | F     | M     | M     |
| 03    | 04    | 05    | 06    | 07    | 08    | 09    |

Description of new locus haplotypes for this probe-enzyme
Constant fragments (if not already described):

Variable fragments for each new haplotype:

| | |
|---|---|
| Haplotype #14 = | 6.6 , 3.7 |
| Haplotype #15 = | 10.2 , 3.3 |
| Haplotype #17 = | 6.4, 4.6, 3.7 |
| Haplotype #18 = | 10.2, 4.1 |
| Haplotype #19 = | 6.6, 4.2, 3.7 |
| Haplotype #20 = | 6.6, 3.7, 3.6 |
| Haplotype # | |
| Haplotype # | |

Comments:

Table 4E

# TABLE 4 E

**PROBE: LAM4159 ENZYME: P  KINDRED #1421  PERSON/DATE: VB**

Pedigree diagram:
- Circle (0) — Square (0)
- Circle 2,6 (10) — Square 7,8 (9)
- Circle 1.7 (0 2) — Square 6,8 (01)
- Diamonds: 6,7 F 03 | 1,6 F 04 | 6,7 F 05 | 1,6 F 06 | 1,8 F 07 | 7,8 M 08

Description of new locus haplotypes for this probe-enzyme
Constant fragments (if not already described):

Variable fragments for each new haplotype:

| Haplotype# | | | |
|---|---|---|---|
| Haplotype# 8 | 6.0 | 4.2 | 1.9 |
| Haplotype# | | | |
| Haplotype # 1 | 6.0 | 5.4 | 1.9 |
| Haplotype # 6 | 6.0 | 4.2 | 1.95 |
| Haplotype# 7 | 6.0 | 4.8 | 2.3 |
| Haplotype# | | | |
| Haplotype# | | | |
| Haplotype# | | | |

Comments:

Table 4F

**0221633**

# TABLE 4 F

PROBE: λ4-966    ENZYME: R    KINDRED #1331    PERSON/DATE:

Definition of locus haplotype for this probe-enzyme:

Constant fragments:

Variable fragments for each new haplotype:

| Haplotype# 1 = 5.1 |
| Haplotype# 2 = 3.7 |
| Haplotype # 3 = 3.3 |
| Haplotype # 4 = 3.0 |
| Haplotype# 5 = 2.6 |
| Haplotype# 6 = 5.3 |
| Haplotype # 7 = 4.3 |
| Haplotype# 8 = 3.2 |

Comments:

Table 4G

# TABLE 4 G

0221633

PROBE: λ4-368    ENZYME: H    KINDRED #1418    PERSON/DATE:

---

Description of new locus haplotypes for this probe-enzyme
Constant fragments (if not already described):

Variable fragments for each new
haplotype:

| | |
|---|---|
| Haplotype# 4 = 16 | |
| Haplotype# 2 = 11.5 | |
| Haplotype # | |
| Haplotype # | |
| Haplotype# | |
| Haplotype# | |
| Haplotype# | |
| Haplotype# | |

Comments:

0221633

Table 4H

0221633

# TABLE 4H

PROBE: λ4-744    ENZYME: R    KINDRED #1345    PERSON/DATE:

---

Description of new locus haplotypes for this probe-enzyme
Constant fragments (if not already described):

| Variable fragments for each new haplotype: | |
|---|---|
| | Haplotype # 1 = 4.4 |
| | Haplotype # 4 = 3.0 |
| | Haplotype # 6 = 2.5 |
| | Haplotype # |
| | Haplotype # |
| | Haplotype # |
| | Haplotype # |
| | Haplotype # |

---

Comments:

0221633

Table 4I

# TABLE 41

PROBE: λ4-1065  ENZYME: R   KINDRED #1345   PERSON/DATE:

Description of new locus haplotypes for this probe-enzyme
Constant fragments (if not already described):

Variable fragments for each new haplotype:

| Haplotype | | Value |
|---|---|---|
| Haplotype# 1 | = | 5.4 |
| Haplotype# 2 | = | 4.0 |
| Haplotype # 6 | = | 5.6 |
| Haplotype # 11 | = | 6.8 |
| Haplotype # 13 | = | 5.5 |
| Haplotype# 14 | = | 4.4 |
| Haplotype# 15 | = | 4.2 |
| Haplotype# | | |

Comments:

Table 4J

0221633

# TABLE 4 J

PROBE: λ4-962   ENZYME: M   KINDRED#1331   PERSON/DATE:

---

Definition of locus haplotype for this probe-enzyme:

Constant fragments:

Variable fragments for each new haplotype:

| | |
|---|---|
| Haplotype# 1 = 3.7, 2.0, 1.7 | |
| Haplotype# 3 = 4.3, 2.3, 1.7 | |
| Haplotype # 4 = 3.7, 2.3, 1.8 | |
| Haplotype # | |
| Haplotype# | |
| Haplotype# | |
| Haplotype# | |
| Haplotype# | |

Comments:

0221633

Table 5

## Genotyping Strategy

Table 6A

Bone Marrow Transplant Genotyping:  FFLP Probe Panel Applied to 10 Cases

Informativeness of Probes

| Patient/Donor Pair Number | Patient & Donor | Patient | Donor | Not Informative |
|---|---|---|---|---|
| 1. | 1214 159 355 | | 123 962 | 427 966 368 744 1065 |
| 2. | 427 123 | 159 1214 | 355 962 | 966 368 744 1065 |
| 3. | 1214 1065 159 123 | 355 | | 966 368 744 1065 427 |
| 4. | 1214 1065 427 | 962 | 355 | 123 159 966 368 744 |
| 5. | 1065 159 123 | 962 | | 427 1214 355 966 368 744 |
| 6. | 427 355 1214 123 | | 1065 | 159 966 368 744 962 |
| 7. | 427 355 1214 | 123 | | 159 966 368 744 1065 962 |

Table 6A (continued)
Patient/Donor

| Pair Number | Patient & Donor | Patient | Donor | Not Informative |
|---|---|---|---|---|
| 8. | 1065 962 427 | | 1214 | 123 355 159 966 368 744 |
| 9. | 123 962 | | 427 | 1214 355 159 966 368 744 1065 |
| 10. | 1214 | 1065 | 123 427 | 355 159 966 368 744 962 |

33

**0221633**

Table 6B

Probe Information

| Probe | Enzyme | PIC (est.) | Patient and Donor | Patient Only | Donor Only | Not Informative |
|---|---|---|---|---|---|---|
| Lam4-427 | RsaI | 0.95 | 5 | 0 | 2 | 3 |
| Lam4-1214 | BglII | 0.94 | 6 | 1 | 1 | 2 |
| Lam4-355 | BglII | 0.90 | 3 | 1 | 2 | 4 |
| Lam4-159 | PstI | 0.90 | 3 | 1 | 0 | 6 |
| Lam4-123 | RsaI | 0.9 | 5 | 1 | 2 | 2 |
| Lam4-966 | RsaI | 0.85 | 4 | 2 | 1 | 3 |
| Lam4-744 | RsaI | 0.8 | 3 | 4 | 0 | 3 |
| Lam4-1065 | RsaI | 0.75 | 4 | 2 | 1 | 3 |
| Lam4-962 | MspI | 0.70 | 2 | 2 | 2 | 4 |

Table 7

A

B

Lam4-427 · Rsal

Lam4-123 · Rsal

Table 8

Lam4 – 123 · RsaI

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 303 260 (P.A. PETERSON et al.)<br>* complete * | 1,3,5, 7-9,21 | C 12 Q 1/68 |
| X | EP-A-0 084 796 (CETUS CORP.)<br><br>* complete * | 1,5,7- 9,21 | |
| X,D | PROC. NATL. ACAD. SCI. USA, (GENETICS), vol. 77, no. 11, November 1980, pages 6754-6758, Baltimore, US; A.R. WYMAN et al.: "A highly polymorphic locus in human DNA"<br>* page 6756, column 1, paragraph 4 - column 2, paragraph 2 * | 1,5,7- 9,21 | |
| X | NATURE, vol. 306, 17th November 1983, pages 234-238, London GB; J.F. GUSELLA et al.: "A polymorphic DNA marker genetically linked to Huntington's disease"<br>* page 236, figures 2, 3 * | 1,5,7- 9,21 | TECHNICAL FIELDS SEARCHED (Int. Cl.4)<br><br>C 12 Q 1/00<br>G 01 N 33/00<br>C 07 H 21/00<br>C 12 N 15/00 |
| X,P | WO-A-8 602 101 (BIOTECHNICA LTD.)<br>* claims 1-3 * | 1,5,7- 9,21 | |

-/-

The present search report has been drawn up for all claims

| Place of search | Date of completion of the search | Examiner |
|---|---|---|
| BERLIN | 15-12-1986 | GREEN C.H. |

European Patent Office

EP  86 30 6013

## EUROPEAN SEARCH REPORT

| DOCUMENTS CONSIDERED TO BE RELEVANT | | | Page  2 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X,D | NATURE, vol. 313, 10th January 1985, pages 101-105, London, GB; R. WHITE et al.: "Construction of linkage maps with DNA markers for human chromosomes" * complete * | 1,5,21 | |
| X | GB-A-2 135 774 (ACTAGEN INC.) * page 1, lines 6-9; page 4, paragraphs 3, 4; pages 16-19; examples VI-IX, claims * | 1,5,7-9,21 | |
| A | CLINICAL CHEMISTRY, vol. 31, no. 6, June 1985, pages 804-811, Washington, US; C.D. SAULS et al.: "Applications of recombinant DNA to pathologic diagnosis" | | TECHNICAL FIELDS SEARCHED (Int. Cl.4) |

The present search report has been drawn up for all claims

| Place of search | Date of completion of the search | Examiner |
|---|---|---|
| BERLIN | 15-12-1986 | GREEN C.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82